(19)

**Europäisches Patentamt**

**European Patent Office**

**Office européen des brevets**

(11) **EP 3 682 937 B1**

(12) **EUROPEAN PATENT SPECIFICATION**

(45) Date of publication and mention of the grant of the patent:
**29.11.2023 Bulletin 2023/48**

(21) Application number: **20162011.9**

(22) Date of filing: **06.05.2016**

(51) International Patent Classification (IPC):
**A61M 25/10** *(2013.01)*    **A61B 1/00** *(2006.01)*
**A61B 5/00** *(2006.01)*

(52) Cooperative Patent Classification (CPC):
**A61B 5/0066; A61B 1/00082; A61B 1/00097; A61B 1/00148; A61B 1/00172; A61B 1/00194; A61B 5/6853;** A61M 25/10

(54) **AUTOMATIC INFLATION/DEFLATION DEVICE APPLIED IN OCT ENDOSCOPIC SCANNING AND IMAGING SYSTEM**

AUTOMATISCHE INFLATIONS-/DEFLATIONSVORRICHTUNG VERWENDET IN EINEM ENDOSKOPISCHEN OCT-ABTASTUNGS- UND -BILDGEBUNGSSYTEM

DISPOSITIF DE GONFLAGE/DÉGONFLAGE AUTOMATIQUE APPLIQUÉ DANS UN SYSTÈME DE BALAYAGE ET D'IMAGERIE OCT ENDOSCOPIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.05.2015 CN 201510234799**

(43) Date of publication of application:
**22.07.2020 Bulletin 2020/30**

(62) Document number(s) of the earlier application(s) in accordance with Art. 76 EPC:
**16792139.4 / 3 295 987**

(73) Proprietor: **Micro-Tech (Nanjing) Co., Ltd.**
**Nanjing, Jiangsu 210000 (CN)**

(72) Inventors:
• **LIU, Hui**
**Nanjing, Jiangsu 210000 (CN)**
• **XI, Jiefeng**
**Nanjing, Jiangsu 210000 (CN)**

• **GAO, Duangui**
**Nanjing, Jiangsu 210000 (CN)**
• **CHANG, Jian**
**Nanjing, Jiangsu 210000 (CN)**
• **WANG, Jiwei**
**Nanjing, Jiangsu 210000 (CN)**
• **ZHANG, Liting**
**Nanjing, Jiangsu 210000 (CN)**
• **LI, Changqing**
**Nanjing, Jiangsu 210000 (CN)**
• **LENG, Derong**
**Nanjing, Jiangsu 210000 (CN)**

(74) Representative: **Kolster Oy Ab**
**Salmisaarenaukio 1**
**P.O. Box 204**
**00181 Helsinki (FI)**

(56) References cited:
**WO-A1-2009/032016**    **WO-A1-2012/029685**
**US-A1- 2004 220 522**    **US-A1- 2010 004 544**

## Description

### Field of the Invention

The present invention relates to an automatic inflation/deflation device. Specifically, the invention relates to the application of automatic inflation/deflation device in optical coherence tomography endoscopic scanning and imaging system.

### Description of the Related Art

Optical coherence tomography (OCT) has been widely used in the field of ophthalmic diagnosis. This technology is based on the optics, electronics and computer science and technology. It's a new imaging technology combining photoelectricity, high-speed data acquisition, image processing and other advanced disciplines. OCT has attracted much attention because of its high resolution, high speed imaging and so on, it's already been applied in biomedical and clinical diagnosis.

Compared to the CT, ultrasound, MRI and the others, OCT has a very high resolution. Also compared to the traditional laser confocal microscopy, the imaging depth of OCT has obvious advantages. Most of the core technologies of traditional optical probes is using fiber bundle for light transmission and imaging, or using CCD technology for imaging, nevertheless such endoscope probe can only detect the lesion on the surface of tissue, but the symptoms of early cancer usually occurred under the skin below the depth of 1-3 mm, so that the traditional optical endoscope probe seems unavailable. At present, there are endoscope probes for medical imaging by means of the ultrasound, the deep tissue information about the biological tissue under the surface can be obtained, but the resolution is only in the order of millimeters, which is easy to cause a misdiagnosis of early cancer.

With the OCT technology developing over the past decade, the endoscopic OCT technology is born and develops as a branch of the OCT technology. The core goal of the endoscopic OCT technology is to miniaturize the device without reducing the resolution, and to provide high resolution OCT image for the internal organs of human body. This technology greatly extends the application of OCT technology, making OCT inspection objects have been involved in a variety of lumens in digestive system, such as large digestive tract lumen (e.g. esophagus, rectum) and smaller digestive tract lumen (e.g. biliary tract) and so on.

Nowadays, when the OCT is used for scanning the patients' esophagus, biliary tract, intestinal tract and the others, doctors have to manually perform the inflating/deflating operations of the balloon that is inside the human body. It has many limitations, for example, the inflatable accuracy is hard to be accurately controlled, speeds of inflation and deflation are slow, errors may be occurred during the manual operation of the doctor, there's no over-voltage protection and so on. Therefore, a new technology is urgently needed to resolve these limitations to meet actual medical work.

US 2010/004544 A1 discloses that the inflation/deflation of the different balloons can be controlled by using an air supplying pump and an air supply switching valve.

## SUMMARY OF THE INVENTION

The present invention provides an OCT endoscopic scanning and imaging system according to claim 1. Further aspects and preferred embodiments are defined in the dependent claims. Aspects, embodiments, examples, and methods of the present disclosure that do not fall within the scope of the appended claims do not form part of the invention and are merely provided for illustrative purposes.

One purpose of this invention is to provide an OCT endoscopic scanning and imaging system with an automatic inflation/deflation device, comprising an OCT endoscopic scanning and imaging system and an automatic inflation/deflation device connected to the OCT endoscopic scanning and imaging system, wherein the OCT endoscopic scanning and imaging system comprises a balloon, an inflation catheter, and an OCT microprobe, characterized in that the automatic inflation/deflation device comprises

a control and display module for setting and controlling an inflation/deflation status of the balloon,
an air pump that is connected to the control and display module and is controlled by the control and display module for activation and stop, wherein the air pump is communicated with the inflation catheter for an inflation or deflation of the balloon;
an inflation solenoid valve that is provided in the inflation catheter between the air pump and the balloon for starting/stopping an inflation process, wherein the inflation solenoid valve is further connected to the control and display module so as to be opened/closed by the control and display module;
a deflation solenoid valve that is provided in a bypath of the inflation catheter between the air pump and the balloon for starting/stopping a deflation process, wherein the deflation solenoid valve is further connected to the control and display module so as to be opened/closed by the control and display module; and
a pressure sensor that is communicated with the inflation catheter for collecting pressure data of the balloon, wherein the pressure sensor is connected to the control and display module such that the collected data may be transmitted to the control and display module.

Preferably, the automatic inflation/deflation device further comprises a throttle valve that is provided in the inflation catheter between the inflation solenoid valve and the balloon for controlling an inflation/deflation rate in the inflation catheter.

Preferably, the automatic inflation/deflation device further comprises a mechanical pressure switch that is provided in the inflation catheter between the throttle valve and the balloon for hardware pressure relief protection.

Preferably, the automatic inflation/deflation device further comprises an anti-explosion pressure sensor that is provided in the inflation catheter between the throttle valve and the balloon for detecting air pressure in the balloon.

Preferably, the automatic inflation/deflation device comprises multiple pressure sensors that are provided in the inflation catheter at multiple positions for collecting air pressure data in the balloon at the multiple positions respectively.

Preferably, among the multiple pressure sensors, at least one pressure sensor is provided in the inflation catheter between the inflation solenoid valve and the throttle valve, and at least one pressure sensor is provided in the inflation catheter between the balloon and the throttle valve.

Preferably, the OCT endoscopic scanning and imaging system further comprises a double cavity tube and a handle, wherein the double cavity tube is connected to the handle at one end and to the balloon at the other end; wherein one cavity of the double cavity tube is communicated with the inflation catheter, and the other cavity of the double cavity tube is provided with an inner tube for passing through the OCT microprobe.

Preferably, the inner tube and the balloon are concentrically arranged such that an offset distance between a center of the inner tube and a center of the balloon is of no more than 500 micrometers under 3 atmospheric pressure.

Preferably, the OCT endoscopic scanning and imaging system further comprises an image display module and a data processing module connected to the image display module; wherein the handle is provided with a host interface and a ventilation interface, such that the handle is connected to the data processing module via the host interface, and the air pump is communicated with the inflation catheter via the ventilation interface.

Preferably, the OCT microprobe comprises a single-mode fiber, a spring tube, a glass rod, a self-focusing lens and a reflector; wherein the single-mode fiber is arranged in the spring tube for transmitting light signals and torsion force; one end of the glass rod is surface-glued to the self-focusing lens at a zero angle, and the other end of the glass rod is obliquely surface-glued to the single-mode fiber; wherein the reflector is provided at a side of the self-focusing lens that is far away from the glass rod so as to reflect light singles of the self-focusing lens to change transmission direction of the light signals.

Preferably, the control and display module is set for performing procedures of:

an inflation process that sets an inflation pressure and an inflation time of the balloon, and activates the air pump and the inflation solenoid valve to inflate the balloon until the preset inflation pressure is reached;
a deflation process that sets a deflation pressure and a deflation time of the balloon, and activates the air pump and the deflation solenoid valve to deflate the balloon until the preset deflation pressure is reached; wherein
during the inflation/deflation processes, pressure data within the balloon and the inflation catheter is monitored by the multiple pressure sensors, such that the air pump is turned off when the air pressure exceeds the preset pressure, and/or the pressure is relieved through the mechanical pressure switch.

Another purpose of this invention is to provide an application of an automatic inflation/deflation device in an OCT endoscopic scanning and imaging system, the OCT endoscopic scanning and imaging system comprising: a sweep laser module, an interference module, a probe module, a data collection module, a data processing module, an image display module, an actuator, a balloon catheter, an OCT microprobe, and an automatic inflation/deflation device, the automatic inflation/deflation device comprising a control and display module, an air pump, an inflation solenoid valve, a deflation solenoid valve, a pressure sensor, an explosion-proof pressure sensor and a mechanical pressure switch. The automatic inflation/deflation device is applied in the OCT endoscopic scanning and imaging system to implement automatic inflation/deflation and precise air pressure control, wherein the air pump is connected to the throttle valve through the inflation solenoid valve and the deflation solenoid valve, the throttle value is also connected to the balloon, and at least one pressure sensor is connected to the balloon, at least one pressure sensor is disposed between the throttle valve, the inflation solenoid valve, and the deflation solenoid valve, explosion-proof pressure sensor is connected with the balloon, the mechanical pressure switch is connected with the balloon, the control and display module is capable of setting the balloon air pressure and the inflation time, collecting pressure, controlling the air pump to start and stop, and controlling the working condition of the solenoid valve.

The process of using the automatic inflation/deflation device comprises:

an inflation process: setting the balloon pressure and inflation time on the control and display module, and keeping the pump inflating until the preset pressure value is reached;
a deflation process: setting the balloon pressure and deflation time on the control and display module, and keeping the pump deflating until the preset pressure value is reached; and

in the inflation/deflation processes, the system is under over-pressure protection by the explosion-proof pressure sensor, the air pump is closed and the pressure can be relieved through the mechanical pressure switch when the air pressure of the balloon exceeds the preset pressure value.

The automatic inflation/deflation device accomplishes automatic inflating and deflating. It has a function of setting different air pressure parameters, and can perform inflation/deflation on balloons of different specifications, the inflation process can be stopped automatically when the balloon pressure reaches the preset value, and it has over-voltage protection function.

The other purpose of this invention is to provide an OCT endoscopic scanning and imaging system comprising: a sweep laser module, an interference module, a probe module, a data collection module, a data processing module, an image display module, an actuator, a balloon catheter, an OCT microprobe, and an automatic inflation/deflation device.

The sweep laser module comprises a high-speed sweep laser, a fiber isolator, a fiber couple, it stops the optical signal from the sweep laser and the subsequent optical path to prevent the optical signal of the subsequent optical path from interfering the normal operation of the laser; the interference module can use fiber-type Mach-Zehnder interferometer (MZI) or fiber-type Michelson (Michelson) interferometer structure. The MZI is mainly composed of two fiber couplers, two fiber circulator and two fiber polarization controller, in which one of the fiber couplers usually use asymmetric fiber coupler to output most of the laser to the microprobe of the laser, a fiber circulator is arranged in both the reference arm and the sample arm to collect optical signals reflected or scattered back from the two arms; the other fiber coupler is a symmetrical 2x2 fiber couple (which means a splitting ratio of 50/50) to produce an interference optical signal and to reduce the DC common mode signal, the fiber polarization controller is symmetrically placed in the reference arm and the sample arm for adjusting the polarization state of the two arms to obtain the best interference optical signal. The Michelson structure is composed of a symmetrical $2 \times 2$ fiber coupler, a fiber circulator and two fiber polarization controllers, the sweep laser passes through the fiber circular and then get into the fiber coupler, the optical signals reflected or scattered back from the reference arm and the sample arm generate interference signal when the sweep laser passes the same fiber coupler, The optical signals reflected or scattered by the arms are generated by interfering with the same fiber coupler. The fiber polarization controllers are symmetrically placed in the reference arm and the sample arm to adjust the polarization state of the two arms to obtain the best interference signal. The advantages of the MZI lie in symmetry structure, simple dispersion management and high detection sensitivity. The advantages of Michelson interferometer lie in the simple structure and without introducing a polarization mode dispersion (PMD); the two interferometers are common in that an optical path difference between two arms determines a free spectral range (FSR) in which a clock signal is generated, and finally determines a maximum imaging depth of an OCT image; the probe module can be a high-speed balanced photodetector, which is mainly used to convert an interference optical signal output from the interference module into an electrical signals; the data collection module is high-speed modulus collection card which is mainly used for converting the analog electrical signal into digital electrical signals, and providing the digital signals to the data processing module for digital signal processing. The data processing module is a chip (such as CPU, GPGPU, DSP, FPGA, etc.) with digital signal processing capability, which is mainly used for processing the original signals and transferring them into the final image signals; and the image display module is mainly used for displaying the image signal and the post-processing and measurement of the image; the actuator is composed of an fiber rotating connector, a motor and an electric displacement platform, the rotating motor inside the actuator drives the OCT microprobe to rotate scanning, at the same time the electric displacement platform drives the actuator to move in a certain direction, the software reconstructed the acquired rotation scan data and the translation table movement data to a 3D image;; the OCT microprobe is mainly used to enter the internal organs of the human body to transfer sweep laser and collect the optical signals backscattering from the biological tissue; the balloon catheter is used for expanding the lumens from the internal organs of the body, and eliminating wrinkles and stabilizing the OCT microprobe to the center of the balloon; the automatic inflation/deflation device is mainly used for dilation of balloon catheters. By using an automatic inflation/deflation device in an OCT endoscopic scanning and imaging system has the effects like: first of all, the manual operation of a doctor inflating/deflating a balloon is omitted, thereby shortening the inflating/deflating time for the doctor to improve the safety and avoid the risk of explosion caused by over-inflation of the balloon; secondly, second, the precise air pressure control balloon guarantees the inflatable shape consistency, since the optical imaging is sensitive to the shape of the scanned object uplifted by the balloon, the repeatability of the multiple scans for the same object is good, the doctor is able to compare the scanned image data; what's more, in the case of emergency treatment, the doctor can perform other operations while the automatic deflation is performed.

Preferably, the OCT microprobe comprises a spring tube, which is capable of providing sufficient torque to keep both the distal and proximal tips of the probe with a certain length operating synchronously when rotating, and the single-mode fiber is transmitting inside the spring tube; a lens assembly comprises a glass rod and a self-focus lens, making the light from the optical fiber gathering outside the preset working distance, the working distance of the OCT microprobe can be changed by changing the bonding distance between the glass rod and the single-mode fiber, and the lateral resolution of the OCT probe can be improved at the same time; the clear aperture of the self-focus lens can be increased by the bonding between the self-focus lens and the glass rod, and then the numerical aperture and lateral resolution of the OCT probe can be increased, and the physical size of the probe will be optimized. The OCT microprobe can also include a speculum, a support stainless steel tube and a grooved stainless steel tube, the tip faces of which are bonded with an optical glue.

Wherein, one side of the single-mode fiber is a standard fiber splice, this splice can connect to the rotating end of the optical fiber end in the OCT system, the single-mode fiber is inside the a spring tube (covered with a PTFE film), the spring tube can protect the single-mode fiber effectively and reduce the resistance when the probe is rotating, so that the OCT microprobe scans more smoothly, the standard fiber splice combines a support stainless steel tube, the stainless steel tube supports the OCT microprobe for scanning to make the entire rotating and scanning process more smooth. The other side of the single-mode fiber is an incline and is glued to one end face of the glass rod which is also an incline, the inclination of the gluing face reduces the interference of the reflected light to the signal light effectively, and the working distance of the OCT microprobe reaches the expected working distance by changing the bonding distance between the glass rod and the single-mode fiber. And the other end of the glass rod is glued with the self-focus lens at the angle 0°end and then sealed inside the grooved stainless steel tube; by changing the length of the self-focus lens, the working distance of the OCT probe is changed to obtain the best lateral resolution with the specified working distance, and the numerical aperture of the OCT probe can be increased by increasing the length of the glass rod, thereby the lateral resolution is increased as well, that means the usage of the glass rod not only increases the working distance of the microprobe, but also increases the numerical aperture of the microprobe, and the increase of the numerical aperture leads to the increase of the lateral resolution, at the same time the length of self-focus lens is also greatly reduced , and the micro-probe's bend is guaranteed so that the entire micro-probe can enter the human esophagus directly through the endoscope clamp and the catheter. The reflector can turn the forward light sideways, and the reflector is a cylindrical mirror, which could change the astigmatism influence of the protective cannula outside the OCT probe on the aggregated beam. The self-focus lens is glued to the glass rod, wherein the surface of the self-focus lens in contact with air is coated with an antireflection film, which could reduce the light reflection between the optical surface and increase the light transmission ability, thereby reducing the influence of the reflected light from the optical surface on the signal light and improving the sensitivity of the OCT microprobe as well, the surface of the self-focus lens in contact with air can be machined to a 4°-8° incline to further reduce the interference single of the light through the surface. An angle of an inclined plane at which the single-mode fiber and the glass rod are glued is 4°-12°. A reflective plane of the reflector faces a notch of a stainless tube and is encapsulated in the stainless tube. To reduce the impact on imaging of light scattering of a light source passing through a cylindrical inner tube, the reflector herein can be a cylindrical reflector according to the inner and outer diameters of the cylindrical inner tube and the refractive index of the inner tube material. After the reflector is added, the reflector can reflect and change optical paths of the incident rays In addition, a specially designed mirror of the reflector converges the light rays, so as to offset light scattering impact of the inner tube and correct a shape of a facula, thereby improving the imaging quality.

Preferably, the balloon catheter includes: a handle, where one interface of the handle is a host interface, and the other interface is a ventilation interface; a double-cavity tube, allowing an OCT optical probe to pass through; a balloon, where a front end of the balloon is blocked, and scales are provided on the balloon; an inner tube, where the length of the inner tube is determined according to the length of the balloon, and the length is less than that of the balloon; when the balloon is welded with a soft head, the balloon is pushed downwards for a certain distance, so as to be aligned with and fixed to the inner tube and then to be welded, the definition of the scanning image can be affected if the inner tube is too thick, and the rotation of the probe and concentricity can be affected if the inner tube is too thin; the inner tube is exclusively designed for the OCT probe; the inner diameter of the inner tube is 1.4 mm, and the outer diameter is 1.65 mm; the concentricity between the inner tube and the balloon is an offsets of no more than 500 micrometers under rated working atmospheric pressures, preferably, the rated working atmospheric pressure is 3 to 5 atmospheric pressures, and the concentricity between the inner tube and the balloon is an offset by no more than 500 micrometers under 3 atmospheric pressures; a film sleeve, located at a junction between the balloon and the double-cavity tube and control the inner tube to float within a lumen, so as to ensure that the inner tube does not offset from the center of the balloon; and a soft head, which is a solid structure, where one end of the double-cavity tube is connected to the handle, the other end is connected to the inner tube and the balloon, and the other end of the balloon and the other end of the inner tube are connected to the soft head.

A conventional balloon catheter needs to be supported and guided by a guide wire. The diameter of the guide wire is usually 0.018 in, 0.035 in, 0.014 in, and 0.038 in. The balloon catheter in the present invention can pass through a 0.055 in OCT optical microprobe. Ink printing scales whose line width is less than or equal to 0.1 mm are provided on the balloon, so that a scanning direction of the microprobe can be recognized. This does not affect scanning judgment of a normal image and can also recognize a scanning position on a display screen. The front end of the balloon is blocked to prevent the entrance of body fluid, so that the impact of the body fluid on optical scanning can be prevented, and corrosion by the body fluid on precise instrument can also be prevented. In addition, the blocking material is a soft structure and does not scratch tissues and tracts of the probed object in an operation process, thereby increasing the security of the device. The soft head is a slid structure and can prevent the entrance of the body fluid.

Preferably, the rated working atmospheric pressure is 3-5 atmospheric pressures.

Preferably, the rated working atmospheric pressure is 3 atmospheric pressures, and a concentricity between the inner tube and the balloon is an offset of no more than 500 micrometers under 3 atmospheric pressures. The working rated pressure of the balloon is 3 atmospheric pressures, and a normal esophagus will not be destroyed destroy a normal esophagus under a relatively low pressure. In addition, the heat setting process and the welding process of the balloon can ensure that the concentricity between the inner tube and the balloon is an offset of no more than 500 micrometers under three rated atmospheric pressures, so as to facilitate optical imaging.

Preferably, the balloon catheter includes a film sleeve, located at a junction between the balloon and the double-cavity tube.

The double-cavity tube is connected to the handle by using a UV adhesive, and all the other parts are connected by using a welding process. The length of the inner tube is determined according to the length of the balloon, and the length is shorter than that of the balloon; when the balloon is welded with a soft head, the balloon is pushed downwards for a certain distance, so as to be welded after being aligned with and fixed to the inner tube, and then the balloon has an elongation allowance when the balloon is filled, so as to match stretching of the inner tube and keep concentric. The welding way to fix the balloon can avoid, to some degree, a problem that an optical probe is eccentric caused by relative movement of the catheter and the balloon when a patient and an organ cavity moves and a catheter is excessively inserted into the balloon in a structure in which the inner tube is not fixed and is directly inserted into the balloon.

Preferably, a folding and curling temperature for the balloon is 40°C to 45°C, and the shaping time is 4h to 5 h. Compared to a conventional balloon folding process, this process can maintain memory characteristics of the balloon while ensuring the concentricity. In addition, the material of the handle in the present invention can be polycarbonate, the materials of the double-cavity tube and the soft head can be block polyether amide, and the materials of the balloon and the inner tube can be nylon and a modified polymer thereof.

Preferably, the OCT endoscopic scanning and imaging system includes an optical clock module consisting of the interference module, the probe module, and an optical clock conversion circuit module. The interference module can be an all-fiber Mach-Zehnder Interferometer (MZI) structure which is mainly consists of two fiber couplers. A second coupler is a symmetric 2×2 fiber coupler. First of all, light is divided into two paths at the first fiber coupler, these two paths of light respectively pass through a first fiber and a second fiber that have a fixed optical path difference, interference occurs at the second fiber coupler. The probe module can be a high-speed balanced photodetector which is mainly used to convert an interference optical signal output from the interference module into an electrical signal. The optical interference signal generated by the MZI is converted into an electrical signal by the balanced photodetector, and then converted into an optical clock signal that is uniform in the frequency domain and frequency-variable in time domain by an optical clock conversion circuit module, that is, sequentially passes through a wideband 90° phase shifter, a zero-crossing comparator, an exclusive-OR gate, an OR gate, and an optical clock signal output module to be converted into an optical clock signal that is uniform in the frequency domain and frequency-variable in time domain. The wideband 90° phase shifter is mainly used to shift a phase of an MZI electrical signal by 90 degrees, so as to increase an available spectrum interval width of an original signal, enrich spectrum distribution resources when taking sample of clock signal, and optimize the obtained clock signal sample. The zero-crossing comparator is mainly used to perform zero crossing comparison on the original MZI electrical signal and the phase-shifted MZI electrical signal to convert them into digital signals, and zero points of the MZI signal are uniformly distributed on a frequency domain. Therefore, rising edges or falling edges of the digital signals generated after zero crossing comparison are also uniformly distributed on a frequency domain. The exclusive-OR gate is mainly used to merge two digital clock signals to generate two clock signals in a free spectral range (FSR). In this way, a maximum imaging depth of OCT is increased without increasing the FSR, and jitter generated by the optical signal is reduced as well. In addition, because a sweep laser always has some idle time between two adjacent times of scanning, the optical clock signal needs to fill in some fake clock signals in the blank by using an OR gate to ensure that a high-speed analog-digital capture cards can work normally. The OR gate implements a function of merging real optical clock signals and fake clock signals. The optical clock signal output module is mainly used to transport the merged actual optical clock signals and real clock signals to the data collection module. By using the optical clock module in the OCT endoscopic scanning and imaging system, the demand of a data collection and processing system can be reduced, the collection of redundant information is reduced, and the burden of a storage system is relieved, so as to improve the integration level of the whole OCT system and to reduce the cost of the system. In addition, the signal-to-noise of an image signal can also be improved, and the attenuation of the detection sensitivity is reduced, so as to improve the definition of the image.

Preferably, a method for processing an OCT signals in an OCT endoscopic scanning and imaging system by using a General Purpose Graphics Processing Unit (GPGPU) includes four steps: (1) data collection; (2) data transmission; (3) data processing; and (4) transferring the data to an image display library.

(1) Data collection: FD-OCT original data is obtained by using an external collection device in the present invention.

(2) Data transmission: the FD-OCT original data obtained in the data collection step is placed in a computer system or an embedded system memory. The data is stored in a system memory in frame as a unit. After a certain condition is satisfied (for example, data accumulates to one frame or multiple frames), the data can be transmitted to a device memory of the GPGPU by using a data bus (such as PCI Express). Because a transmission speed of the bus is relatively slow, at the same time of data transmission, the GPGPU performs parallel processing on the OCT original data which is previously transmitted to the device memory. The method has an efficient parallel signal processing capability, can implement real-time digital signal processing, greatly improves transmission efficiency and saves bus resources.

(3) Data processing: the GPGPU processes the digital signal for three steps: one-dimensional digital resampling, one-dimensional Fast Fourier Transform (FFT), and amplitude calculation and normalization. In the one-dimensional digital resampling step, a quick one-dimensional cubic interpolation is implemented once by linear texture lookup twice to improve the accuracy of resampling.

(4) Transferring the data to an image display library: the processed data is placed in a memory of the image display library; the image display library can directly call the data which does not need to be transmitted by using a bus, thereby greatly improving the transmission efficiency and saving bus resources, providing an efficient parallel signal processing capability, implementing real-time digital signal processing, providing high transplantability, improving the flexibility of software display because of seamless combination with the popular image display library (for example, post-processing can be performed on the image by using the GPGPU), and implementing relatively low cost of hardware and software development.

Another objective of the present invention is to provide a method for using an automatic inflation/deflation device applied to an OCT endoscopic scanning and imaging system. First, a user sets parameters such as a balloon air pressure and an inflation time, issues an inflation command, and closes an air pump until a balloon is inflated to a set air pressure value. Then, first, the user sets parameters such as the balloon air pressure and a deflation time, issues a deflation command, and closes the air pump until the set air pressure value is reached by means of air extraction. In the inflation and deflation processes, the system monitors a feedback value of an anti-explosion pressure sensor in real time; if the feedback value exceeds an air pressure upper limit value set by the user, software program protection is immediately performed. The air pump is closed, and an alarm is given.

By using the automatic inflation/deflation device in the OCT endoscopic scanning and imaging system, an operation of performing manual inflation and deflation on the balloon by a doctor can be omitted, so that time of inflation and deflation performed by the doctor can be shorted. Therefore, the security is improved, and a risk of explosion due to over inflation of the balloon is avoided.

BRIEF DESCRIPTION OF THE DRAWINGS

FIG. 1 illustrates a schematic structural diagram of an automatic inflation/deflation device according to the present invention;
FIG. 2 illustrates a working flowchart of an automatic inflation/deflation device according to the present invention;
FIG. 3 illustrates a schematic diagram of an OCT endoscopic scanning and imaging system including an inflation/deflation device according to the present invention;
FIG. 4 illustrates a physical part diagram of an OCT microprobe according to the present invention;
FIG. 5a and FIG. 5b illustrate enlarged sectional views of key parts of an OCT microprobe according to the present invention;

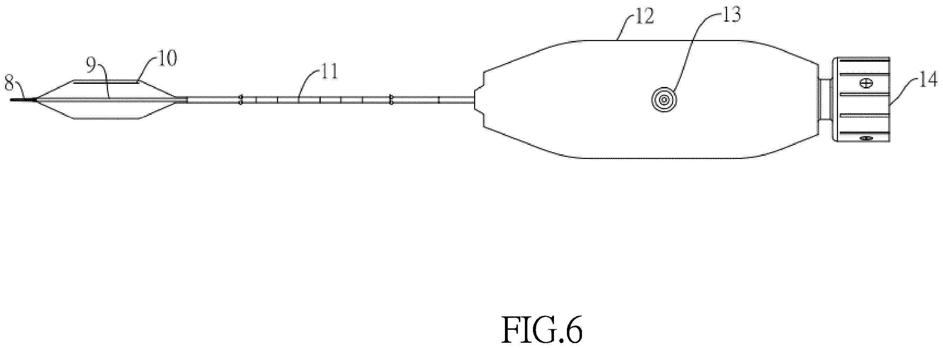

FIG. 6 illustrates a schematic structural diagram of a balloon catheter according to the present invention;
FIG. 7 illustrates a balloon added into a film sleeve;
FIG. 8 illustrates an imaging effect when an eccentricity between an inner tube and a balloon is approximately 500 micrometers;
FIG. 9 illustrates an imaging effect when an eccentricity between an inner tube and a balloon is less than approximately 500 micrometers;
FIG. 10 illustrates an imaging effect when an eccentricity between an inner tube and a balloon is greater than approximately 500 micrometers;
FIG. 11 illustrates a scanning result of the concentricity of a product on the market;
FIG. 12 illustrates a schematic diagram of an optical clock module according to the present invention;
FIG. 13 illustrates a schematic diagram of a process of generating an optical clock signal according to the present invention;

FIG. 14 illustrates a schematic diagram of an OCT endoscopic scanning and imaging system including an optical clock module according to the present invention;

FIG. 15 illustrates processing steps of an FD-OCT signal according to the present invention;

FIG. 16 illustrates a schematic diagram of parallel occurrence of GPGPU data transmission and signal processing according to the present invention;

FIG. 17 illustrates a schematic structural diagram of overall implementation according to the present invention;

FIG. 18 illustrates a curved-line relationship diagram of a working distance, a gluing distance, and a transverse resolution of an OCT microprobe according to the present invention;

FIG. 19 illustrates a scanning diagram of an esophagus of a healthy animal according to the present invention;

FIG. 20 illustrates a partial enlarged view of a scanning diagram of an esophagus of a healthy animal according to the present invention; and

FIG. 21 illustrates a 3D image of an esophagus of a healthy animal according to the present invention.

## Descriptions for reference numerals

1, single-mode fiber, 2, spring tube, 3, glass rod, 4, self-focusing lens, 5, reflector, 6, slotted stainless steel tube, 7, support stainless steel tube, 8, soft head, 9, inner tube, 10, balloon, 11, double-cavity tube, 12, handle, 13, ventilation interface, 14, host interface, 15, film sleeve,

81, scaly epithelial (SE) layer, 82, lamina propria (LP) layer, 83, muscularis mucosa (MM), 84, submucosa (SM), 85, muscularis propria (MP) layer,

91, MZI electrical signal on which 90° phase shift is performed after passing through a wideband 90° phase shifter; 92, MZI electrical signal on which phase shift is not performed, 93, digital signal generated after the signal 91 performs zero crossing comparison, 94, digital signal generated after the signal 92 performs zero crossing comparison, 95, fake clock signal, 96, signal generated after the digital signals 93 and 94 are merged by using an exclusive-OR gate,

101, air pump, 102, inflation solenoid valve, 103, pressure sensor, 104, throttle valve, 105, pressure sensor, 106, anti-explosion pressure sensor, 107, deflation solenoid valve, 108, mechanical pressure switch.

## Detailed description of the preferred embodiments

The technical solutions of the present invention are described in detail below with reference to the accompanying drawings.

Embodiment 1

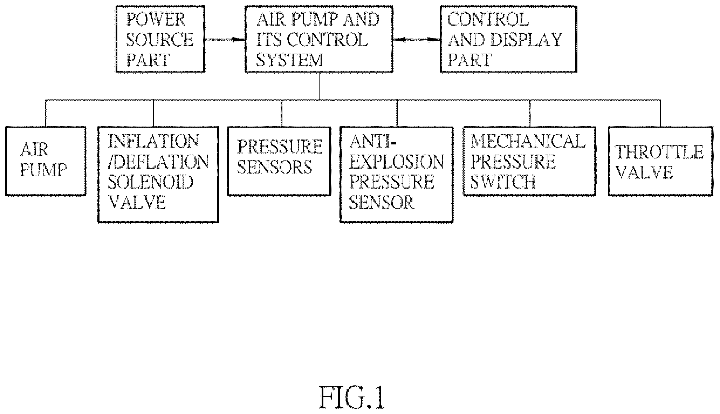

In an automatic inflation/deflation device applied to an optical coherence tomography(OCT) scanning system, the OCT system includes a sweep laser module, an interference module, a probe module, a data collection module, a data processing module, an image display module, an execution mechanism, an OCT microprobe, a balloon catheter, and an automatic inflation/deflation device. As shown in FIG. 1, the automatic inflation/deflation device includes: a power source part, a control and display part, and an air pump and its control system. The air pump and its control system include: the air pump, an inflation solenoid valve, a deflation solenoid valve, a throttle valve, pressure sensors, an anti-explosion pressure sensor, and a mechanical pressure switch.

There are multiple pressure sensors in this application. At least one of the pressure sensors is connected to a balloon. At least one of the pressure sensors is disposed among the throttle valve, the inflation solenoid valve and the deflation solenoid valve. The pressure sensors function to monitor, in real time, a pipeline air pressure and a balloon air pressure during an inflation process and a deflation process. The pressure sensors include two pressure sensors 103 and 105 and form a pressure monitoring system for the inflation process and the deflation process together with multiple solenoid valves in this application. The specific structure is: the inflation solenoid valve 102 and the deflation solenoid valve 107 are provided between the air pump 101 and the pressure sensors 103; the pressure sensor 103 is separated from the pressure sensor 105 by the throttle valve 104; the pressure sensor 105 is connected to the balloon. The following three effects can be achieved by the configuration mentioned above:

(1) Two pressure sensors 103 and 105 are disposed and are separated from each other by the throttle valve 104. Then, the pressure sensor 105 is directly connected to the balloon, and data obtained is more accurate. The pressure sensor 103 not only can monitor the pipeline air pressure for checking an air pressure of the pressure sensor 105, but also can implement a function of early warning and monitoring of inflation and deflation pressures of the air pump after the inflation solenoid valve 102 and the deflation solenoid valve 107 are opened and before the throttle valve 104 is opened during the inflation process and the deflation process. The pressure sensor 103 can close the solenoid valves in time by means of automatic control if there is a problem about the pressures, so as to prevent excessively strong inflation and deflation pressures from causing unnecessary injuries to the balloon or even body cavities of a patient, thereby greatly enhancing the safety of the system.

(2) The pressure sensors 103 and 104 are both connected to the air pump and the outside by using the solenoid valves, and interference of the air pump and an outside air pressure to pressure monitoring of the pressure sensors is isolated by using the solenoid valves.

(3) Double monitoring is performed on the balloon and the pipeline air pressure. Even if a single sensor fails, the system can still normally work, enhancing the stability of the system.

The pressure monitoring system of this application further includes an anti-explosion pressure sensor connected to the balloon. The anti-explosion pressure sensor functions to monitor the air pressure in real time in the inflation and deflation processes. Data monitored by the anti-explosion pressure sensor is independent from data monitored by the pressure sensors. When the monitored data exceeds a preset air pressure upper limit value, a pressure relief protection is activated. If the monitored data keeps unchanged for a long time, the anti-explosion pressure sensor stops the continued pressurization of the air pump. The pressure monitoring system of this application is divided into the pressure sensors and the anti-explosion pressure sensor. Multi-monitoring for the air pressure is implemented by disposing the multiple sensors, thereby enhancing the stability of the system. In addition, different sensors are disposed at different positions and can perform mutual checking and verification. The solenoid valves are provided between the sensors for isolation, so as to avoid interference of an inflation/deflation device and a pressure relief device on pressure measurement, thereby improving the accuracy of pressure measurement. At last, the pressure monitoring system formed by the multiple pressure sensors together with the solenoid valves can prevent excessively strong inflation and deflation pressures from causing unnecessary injuries to the balloon or even body cavities of a patient based on the automatic control function, thereby enhancing the security of the system.

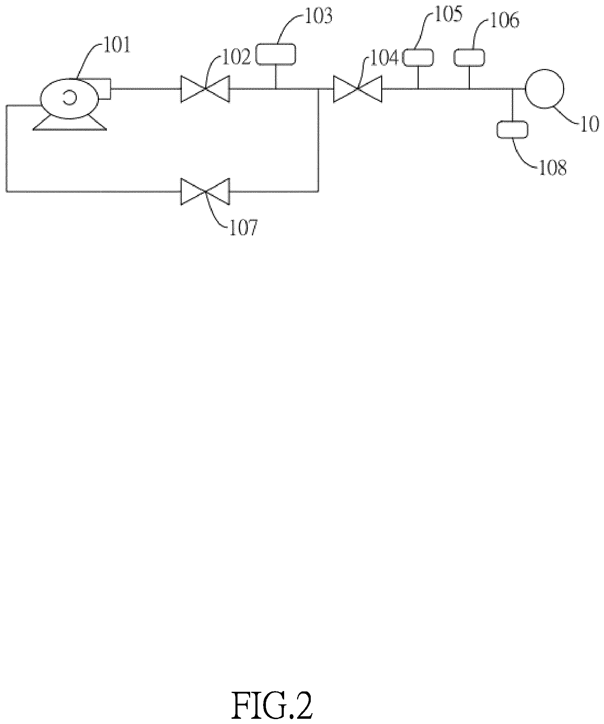

As shown in FIG. 2, a process for using the automatic inflation/deflation device includes:

(1) Inflation process: first, a user sets parameters such as an air pressure and an inflation time of the balloon 10 are set by a user, and issues an inflation command; the control system reads data of the pressure sensors 103 and 105; if the data is less than the air pressure set by the user, the air pump 101 is activated, and the inflation solenoid valve 102 and the throttle valve 104 are opened; feedback values from the pressure sensors 103 and 105 are read in real time in the inflation process; the balloon 10 is inflated until the set air pressure value is reached, and then the air pump 101, the throttle valve 104, and the inflation solenoid valve 102 are closed.

(2) Deflation process: first, the user sets parameters such as the air pressure and a deflation time of the balloon 10; the control system reads data of the pressure sensor 105; if the data is greater than the air pressure set by the user, the air pump 101 is started and the deflation solenoid valve 107 and the throttle valve 104 are opened; a feedback value from the pressure sensor 105 is read in real time in the deflation process; air is drawn from the balloon 10 until the set air pressure value is reached, and then the air pump 101, the throttle valve 104, and then the deflation solenoid valve 107 are closed.

During the inflation and deflation processes, the system monitors a feedback value from the anti-explosion pressure sensor 106 in real time. If the feedback value exceeds an air pressure upper limit value set by the user, software program protection is immediately performed to close the air pump 101 and issue an alarm. The mechanical pressure switch 108 is hardware protection. If the feedback value exceeds the set value, the switch is opened, and pressure relief is performed for protection.

The pressure relief protection is implemented by guiding an entire inflation/deflation device system by means of an automatic control function, is triggered based on the monitoring of the anti-explosion pressure sensor, and is implemented in three ways. First, software protection: when the anti-explosion pressure sensor monitors that the pressure exceeds a limit, the air pump inflation function is disabled and the inflation solenoid valve is also closed; if necessary, the throttle valve and the deflation solenoid valve are opened, and the air pump actively exhausts air to relieve the pressure. In the process, the pressure sensors cooperate to perform pressure monitoring. When the pressure is normal, the solenoid valve and the air pump are closed to and the software protection of pressure relief is stopped, to avoid dangers caused from excessive pressure relief. Secondly, hardware protection: when the anti-explosion pressure switch monitors that the pressure exceeds the limit, the mechanical switch is automatically opened for pressure relief protection. Thirdly, pressure relief valve protection: when the device cannot normally work, a switch on the pressure relief may further be opened to perform manual pressure relief. In this application, three protection ways: software protection, hardware protection, and pressure relief valve protection are provided for the over-pressure risk of the system in cooperation with the anti-explosion pressure sensor, thereby enhancing the safety and the stability of the system and improving the accuracy of the pressure relief system.

In addition, the automatic inflation/deflation device of this application also has the following advantages:

1. A user can set needed parameters, such a pressure value and an inflation time, on a control and display module at anytime, and uploads the parameters in real time to a data system. The system immediately performs monitoring and operation according to new parameters, and can make in-time responses to various accidents during the operation process, thereby enhancing the system security. In addition, accurate air pressure control enables relatively high consistency of the shape of the inflated balloon and higher relevance of obtaining image data for multiple times from a scanned object, facilitating comparison.

2. If the air pressure of the air pump does not change or slightly changes after long-time work, the balloon may be broken or air may be leaked from the balloon. If the air pump continues inflation or air extraction, a body cavity of a patient may be damaged. Therefore, if the pressure monitoring system monitors that the air pressure does not change or slightly changes when the air pump inflates or deflates for a long time, the pressure monitoring system automatically starts a protection mechanism and closes the air pump, thereby enhancing the system security.

3. In this application, the solenoid valves can be quickly opened and closed in an automatic control manner, thereby reducing interference of inflation and deflation on the monitoring system and enhancing the accuracy of the monitoring system.

4. In this application, inflation and air exhaustion are performed in two different channels, and two processes of inflation and deflation can be switched quickly. Especially in the pressure relief protection process, fast air exhaustion can well prevent over-inflation from damaging the balloon and a body cavity of a patient, thereby enhancing the system security.

5. In this application, an automatic inflation and deflation air pump is used, and an inflation pressure range thereof is relatively large. The pressure range is usually controlled in a range from one to five atmospheric pressures according to the body cavity environment used in this application. An air pressure range that can be provided by manual pressure supply is far less than the pressure range in this application.

A power source of the automatic inflation/deflation device is electrified to supply power to the air pump. When the power source is disconnected, the air pump powers off. A main board is controlled to collect pressures, the air pump is controlled to start or stop, and working states of the solenoid valves are controlled. The pressure sensor is in communication with the balloon catheter to monitor a working pressure, and also forms pressure double-insurance monitoring together with an air pump mechanical pressure gauge. If the pressure is exceeded, the solenoid valves are closed and the balloon is enabled to exhaust air. The automatic inflation/deflation device implements automatic inflation and air exhaustion, has a function of setting different air pressure parameters, and can perform inflation and deflation on balloons of different specifications, so as to omit an operation of performing manual inflation and deflation on the balloon by a doctor, and shorten a time of performing manual inflation and deflation on the balloon by the doctor, thereby improving the security and avoiding the risk of explosion due to over-inflation of the balloon. Moreover, the balloon inflatable shape consistency is guaranteed by the precision controlled air pressure c. Because optical imaging is sensitive to the shape of the scanned object supported by the balloon, a doctor can compare image data obtained after performing scanning for multiple times on a same scanned object. The device stops inflation when a set air pressure value is reached in a balloon inflation process and has an over-pressure protection function. When an emergency is processed, the doctor may perform other operations during automatic deflation.

Embodiment 2

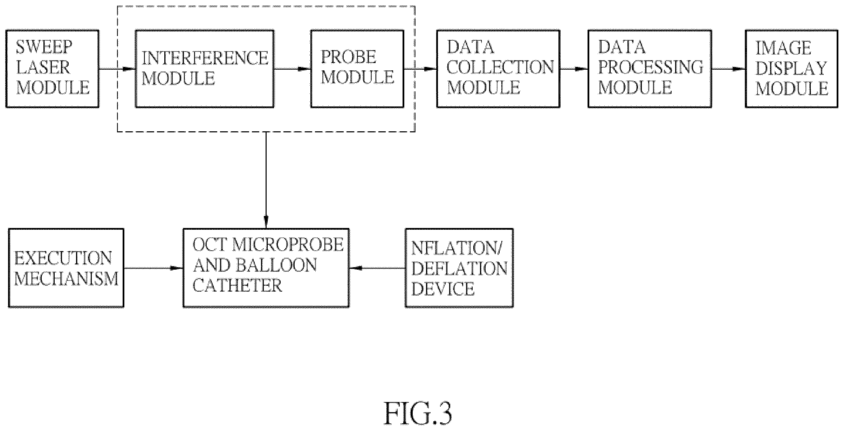

As shown in FIG. 3, an OCT endoscopic scanning and imaging system includes a sweep laser module, an interference module, a probe module, a data collection module, a data processing module, an image display module, an execution mechanism, a balloon catheter, an OCT microprobe, and the inflation/deflation device shown in FIG. 1.

The sweep laser module includes a high-speed sweep laser, a fiber isolator, and a fiber coupler. An optical signal output from the sweep laser is isolated from a subsequent optical path, to prevent an optical signal returned by the subsequent optical path from interfering with normal operation of the laser. The interference module can use a fiber-type Mach-Zehnder interferometer (MZI) or fiber-type Michelson interferometer structure. The MZI is mainly formed by two fiber couplers, two fiber circulators, and two fiber polarization controllers. A first fiber coupler is usually an asymmetric fiber coupler, and outputs most of the laser to a microprobe of a sample arm. The two fiber circulators are respectively placed in a reference arm and the sample arm to collect optical signals reflected or scattered from the two arms. A second fiber coupler can be a symmetric $2\times2$ fiber coupler (that is, the split ratio is 50/50) to generate an optical interference signal and reduce a direct-current common-mode signal. The fiber polarization controllers are symmetrically placed in the reference arm and the sample arm, and are used to adjust polarization states of the two arms to obtain an optimal optical interference signal. The Michelson interferometer structure is formed by a symmetric $2\times2$ fiber coupler, a fiber circulator, and two optical polarization controllers. The sweep laser first passes through the fiber circulator and then enters the fiber coupler. Optical signals reflected or scattered from the reference arm and the sample arm pass through a same fiber coupler and generate an interference signal. The fiber polarization controllers are symmetrically placed in the reference arm and the sample arm and are used to adjust polarization states of the two arms to obtain an optimal optical interference signal. The advantages of the MZI lie in a symmetric structure, simple dispersion management, and high detection sensitivity. The advantages of the Michelson interferometer lie in a simple structure and no introduction of polarized mode dispersion (PMD). The two interferometers are common in that an optical path difference between two arms determines a free spectral range (FSR) in which a clock signal is generated, and finally determines a maximum imaging depth of an OCT image. The probe module can be a high-speed balanced photodetector and is mainly used to convert an interference optical signal output from the interference module into an electrical signal. The data collection module is a high-speed analog-digital collection card and is mainly used to convert an analog electrical signal into a digital electrical signal and provide the digital signal to the data processing module to process the digital signal. The data processing module is a chip (such as a CPU, a GPGPU, a DSP, or an FPGA) having a digital signal processing capability and is mainly used to process an original signal and convert the original signal into a final image signal. The image display module is mainly used to display the image signal and is responsible for post-processing and measurement of the image. The execution mechanism is formed by a fiber rotation connector, a motor, and a motorized translation stage and is mainly used to drive the OCT microprobe to perform mechanical and spiral scanning to obtain an OCT image. The OCT microprobe is mainly used to enter an internal organ of a human body to transmit sweep laser and collect an optical signal scattered backwards from tissues. The balloon catheter is used to expand tracts of internal organs of a human body, eliminate folds, and stabilize the OCT microprobe at the center of the balloon. The inflation/deflation device is mainly used to expand the balloon catheter.

Embodiment 3

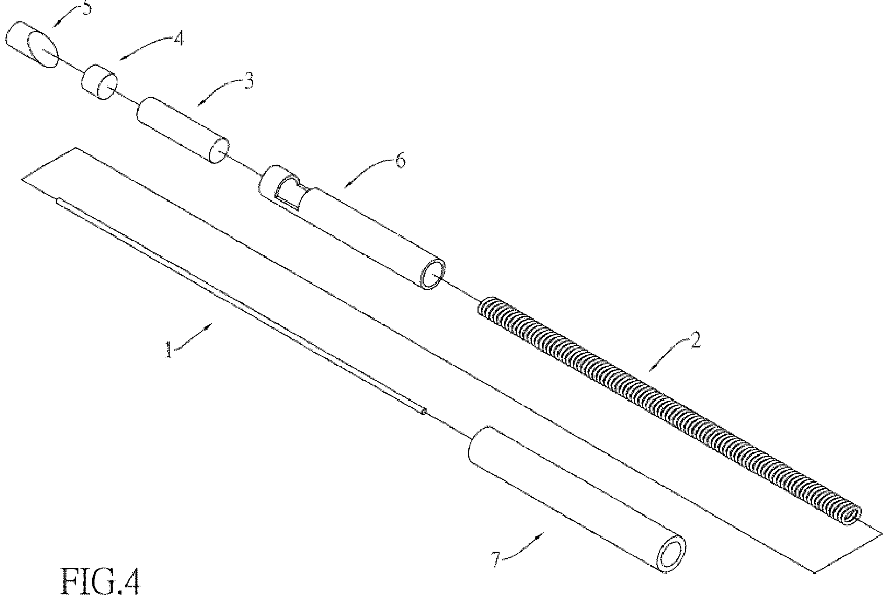

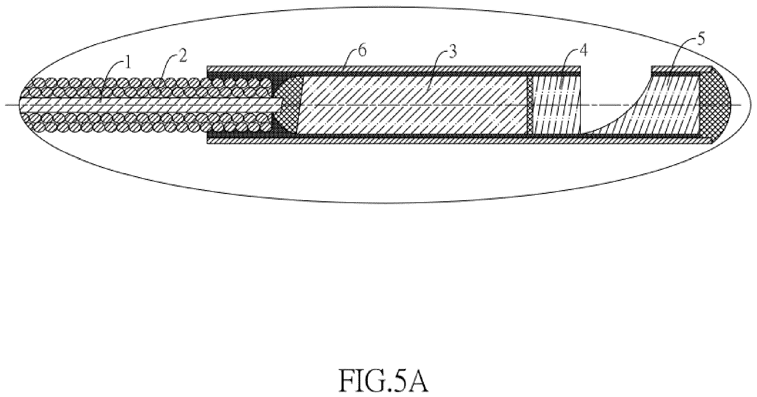

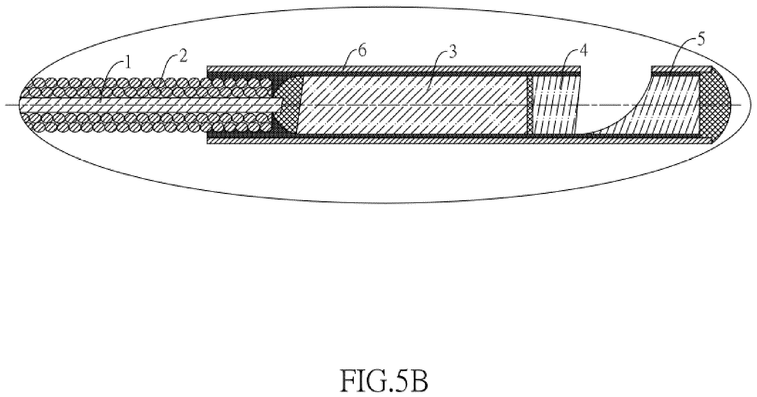

An OCT endoscopic scanning and imaging system is similar to Embodiment 2. The difference between the OCT endoscopic scanning and imaging system and Embodiment 2 is as follows. In the OCT microprobe shown in FIG. 4, FIG. 5a, and FIG. 5b, a single-mode fiber 1 is sleeved in a spring tube 2. The spring tube 2 can provide an enough torsion force, so that a probe of a particular length keeps synchronization between a remote end and a near end during rotation. The spring tube 2 reduces resistance during rotation of the probe while effectively protecting the fragile fiber. One end of a glass rod 3 is surface-glued to a grin lens 4 at a zero angle, and the other end is obliquely combined with the single-mode fiber 1. A working distance of an OCT probe can be changed by changing gluing distances of two end surfaces of the glass rod 3 and the single-mode fiber 1, to achieve a working distance required by expectation, thereby improving the numerical aperture and the transverse resolution of the OCT probe. A reflector 5 is a cylindrical-surface reflector and is encapsulated in a slotted stainless steel tube 6. In addition, a reflective surface of the reflector 5 faces a slotted opening of the slotted stainless steel tube 6, so as to alleviate the impact of light scattering of a light source passing through a cylindrical inner tube on imaging.

As shown in FIG. 4, FIG. 5a, and FIG. 5b, an OCT microprobe of an OCT endoscopic scanning and imaging system includes: a single-mode fiber 1, a spring tube 2, a glass rod 3, a grin lens 4, a reflector 5, a slotted stainless steel tube 6, and a support stainless steel tube 7. End surfaces of these optical elements are glued by using optical adhesives. Specifically, the reflector 5 is disposed into the slotted stainless steel tube 6, and then is placed on working equipment for A/B adhesive dispensing, and then is placed under a microscope for UV adhesive dispensing to assemble the glass rod 3 and the self-focusing lens. The single-mode fiber 1 is inserted into the spring tube 2, and the assembled glass rod 3 and self-focusing lens 4, the single-mode fiber 1, and the spring tube 2 are assembled by means of UV adhesive dispensing. At last, an assembled spring tube assembly is assembled into the slotted stainless steel tube 6, and gaps on edges are filled by an A/B adhesive.

A stainless steel spring tube 2 (coated with a PTFE film) is sleeved outside the single-mode fiber 1 to reduce resistance when the probe rotates while effectively protecting a fragile fiber, so that the microprobe performs scanning more steadily and smoothly. A main function of the support stainless steel tube 7 is supporting when the OCT probe performs scanning, so that the entire probe is steadier when performing rotary scanning. A slot of the slotted stainless steel tube 6 can enable a light beam to be irradiated to a probed sample through the slot.

An antireflective film can reduce reflection of light rays between optical surfaces and enhance light transmission performance, so as to lower the impact of reflective light of the optical surfaces on signal light. Therefore, in this embodiment, by changing the length of the self-focusing lens a working distance of the OCT probe can be changed and an optimal transverse resolution can be obtained under a condition of a specified working distance. An antireflective film is coated on a surface, in contact with air, of the self-focusing lens 4. In addition, the surface, in contact with air, of the self-focusing lens 4 can be processed into a 4° to 8° inclined surface which can further weaken an interference signal of the light rays passing through the surface. FIG. 5a differs from FIG. 5b in: whether the self-focusing lens 4 has a design of an inclination angle of an exit surface. FIG. 5b 2b shows the inclination angle of the exit surface, which is 4° to 8°. This design minimizes the quantity of useless optical signals reflected back by the surface reduced, thereby improving the imaging quality of the OCT probe. In addition, one end of the glass rod 3 is surface-glued to the self-focusing lens 4 at a zero angle, thereby improving the sensitivity and the resolution of the microprobe. The other end of the glass rod 3 is inclined relative to a glued surface of the single-mode fiber 1 by an angle. In this embodiment, the angle of inclination of the glued surface can be 8°. Inclination of the glued surface effectively reduces interference of the reflective light on the signal light. The glass rod 3 is designed and used to increase a numerical aperture of the OCT probe, thereby improving the transverse resolution. In addition, the working distance of the OCT probe can be changed by changing gluing distances of two end surfaces, to achieve a working distance required by expectation. The reflector 5 is installed at an angle of 45°, so that incident light rays are perpendicular to reflective light rays, causing light ray interference. In this embodiment, a 40° reflector 5 is installed on a front end of the microprobe, and the reflector 5 is encapsulated in the slotted stainless steel tube 6, and the reflective surface faces towards the slotted opening of the slotted stainless steel tube 6. In addition, to alleviate the impact of light scattering of the light source passing through the cylindrical inner tube on imaging, the reflector herein in this embodiment is designed as a cylindrical-surface reflector according to the inner and outer diameters of the cylindrical inner tube and the refractive index of the inner tube material. The reflector can change forward light rays into lateral ones, and the reflector is a cylindrical-surface reflector, which can change light scattering impact caused by a protective casing outside the OCT probe on a focused light beam.

Embodiment 4

An OCT endoscopic scanning and imaging system is similar to Embodiment 2. The difference between the OCT endoscopic scanning and imaging system and Embodiment 2 is as follows. As shown in FIG. 4, the balloon catheter includes: a handle 12, where one interface of the handle 12 is a host interface 14, and the other interface is a ventilation interface 13; a double-cavity tube 11, allowing an OCT optical probe to pass through; a balloon 10, where a front end of the balloon 10 is blocked, scales are provided on the balloon, a solid welded structure is blocked on the front end, a blocking socket thereof is disposed within an inner tube, when a device moves within a cavity, the cavity rubs with the inner tube and the balloon and is not in contact with the blocking soft head, and even if the cavity is in contact with the soft head, a friction force tends to press the soft head into the inner tube; an inner tube 9, where the concentricity between the inner tube 9 and the balloon 10 is an offset of no more than 500 micrometers under three rated working atmospheric pressures; a film sleeve 15, the film sleeve is located at a junction between the balloon and a double-cavity tube and controls the inner tube to float within a lumen, so as to ensure that the inner tube does not offset from the center of the balloon; and a soft head 8, which is a solid structure. One end of the double-cavity tube 11 is connected to the handle 12, and the other end is connected to one end of the inner tube 9 and one end of the balloon 10. The other end of the balloon 10 and the other end of the inner tube 9 are connected to the soft head 8. The double-cavity tube 11 is connected to the handle 12 by using a UV adhesive, and all the other parts are connected in a welded manner.

The material of the handle 12 is polycarbonate, the materials of the double-cavity tube 11 and the soft head 8 are block polyether amide, and the materials of the balloon 10 and the inner tube 9 are nylon and a modified polymer thereof.

Ink printing scales whose line width is less than or equal to 0.1 mm are provided on the balloon 10, so that a scanning direction of the probe can be recognized. This does not affect scanning judgment of a normal image and can also recognize a scanning position on a display screen.

Scales are printed on the double-cavity tube 11. Therefore, a doctor can determine a scanning position. A conventional balloon catheter needs to be supported and guided by a guide wire. The diameter of the guide wire is usually 0.018 in, 0.035 in, 0.014 in, and 0.038 in. The double-cavity tube 11 used in the OCT balloon catheter in this embodiment can pass through a 0.055-in OCT optical probe. When optical imaging is performed, a probe light beam emitted out by the probe needs to pass through a wall of the inner wall to reach a probed object. In addition, during imaging, the OCT probe also needs to rotate within the inner tube, so as to comprehensively probe the probed object. In this process, if the wall of the inner tube is excessively thick, because probe light attenuates when passing through the wall of the inner tube, energy loss of probe light is caused, and consequently, imaging is unclear. If the wall of the inner tube is excessively thin, because the OCT probe rotates at a high speed within the inner tube, the excessively thin wall of the inner tube causes a low speed of the probe, and deformation of the inner tube, and consequently, imaging is non-uniform. Therefore, this application defines the inner diameter and the outer diameter of the inner tube, to determine the thickness of the inner tube. In this embodiment, the inner diameter of the inner tube 9 is 1.4 mm, and the outer diameter is 1.65 mm, which are exclusively designed for the OCT probe. The thickness of the inner tube is most appropriate for OCT probe imaging, can ensure that light energy attenuation under this thickness is relatively small, and also ensure strength of the inner tube. A problem of non-uniform and unclear imaging and unclear imaging caused by a low speed of the probe and deformation of the inner tube does not occur.

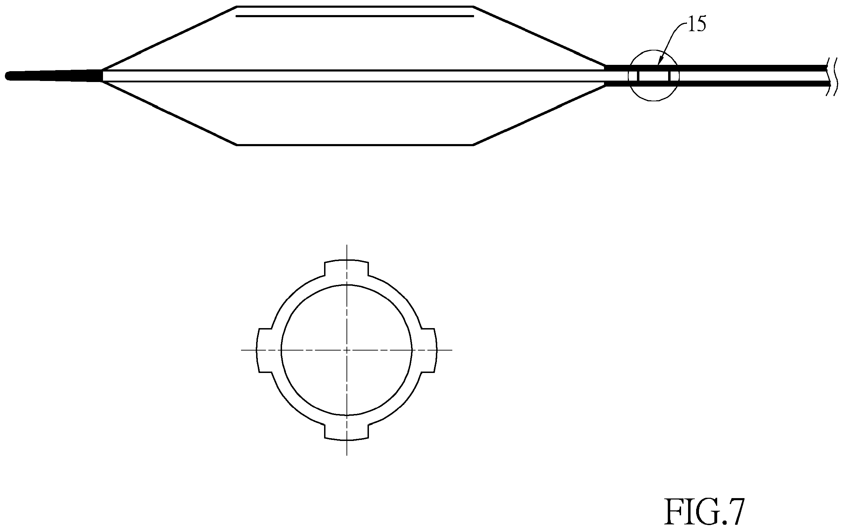

The length of the inner tube is determined according to the length of the balloon, and the length thereof is less than that of the balloon. When the balloon is welded with the soft head, the balloon is pushed downwards by a distance, so as to be aligned with and fixed to the inner tube for welding, so that the balloon has an elongation allowance when the balloon is filled, so as to match stretching of the inner tube and keep concentric. The film sleeve is added to control the inner tube to float within the lumen, so as to ensure that the inner tube does not offset from the center of the balloon, as shown in FIG. 7.

When optical imaging is performed, a tract in which a probed object is located needs to be expanded by the balloon, so as to make imaging complete. If an air pressure is relatively low, folds are formed on the surface of the balloon, and this necessarily affects imaging effects. However, if the air pressure is excessively high, it may damage and destroy the probed object. Therefore, the applicant preferably selects an index of three atmospheric pressures to ensure that the balloon can be completely filled without wrinkles. In addition, the index can also define the length of the balloon and the length of the inner tube. The process of the balloon of this application can ensure that the concentricity between the inner tube and the balloon is an offset of no more than 500 micrometers under three atmospheric pressures, so as to facilitate optical imaging.

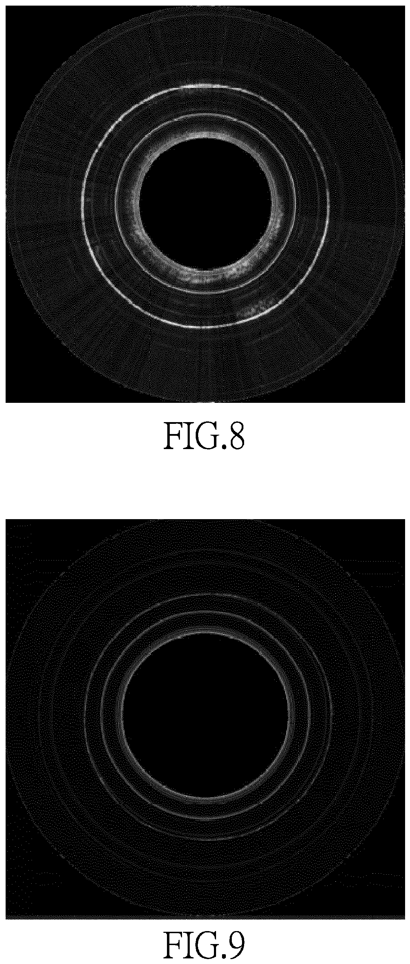

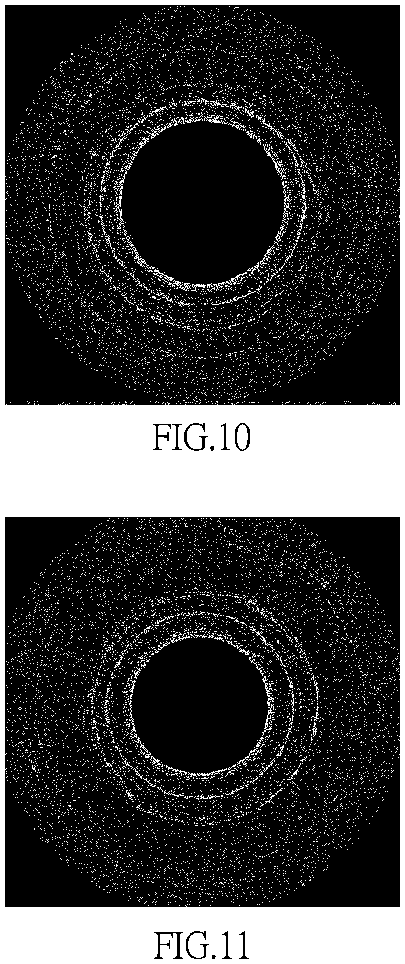

If the concentricity between the inner tube and the balloon is an offset of no more than 500 micrometers, an imaging effect is relatively good. If the offset between the inner tube and the balloon is more than 500 micrometers, a presented image is incompletely displayed. FIG. 8 shows a standard circle whose eccentricity is approximately 500 micrometers. FIG. 9 and FIG. 10 are respectively images that are scanned when the eccentricity of the inner tube and the balloon is less than 500 micrometers and when the eccentricity exceeds 500 micrometers. It can be seen from the foregoing two figures that the eccentricity less than 500 micrometers can meet a requirement of complete imaging. Upon comparison, a general product on the market does not control the index and cannot meet a requirement that the concentricity between the inner tube and the balloon is an offset of no more than 500 micrometers under three atmospheric pressures. A product on the market is selected for contrast, and an imaging effect is shown in FIG. 11. Imaging of an area on the upper right corner is incomplete and an actual scanning requirement cannot be met.

Embodiment 5

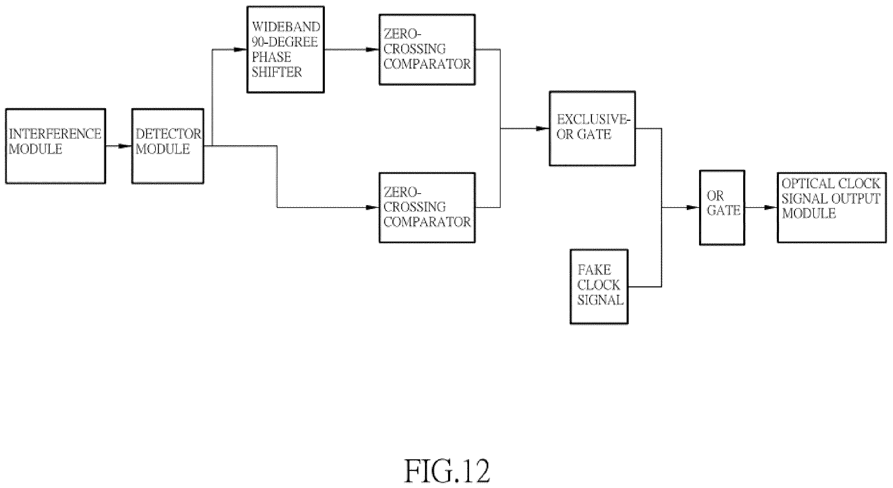

FIG. 12 is an optical clock module using in an OCT endoscopic imaging system, comprising an interference module, a detector module and an optical clock conversion circuit module, the optical clock conversion circuit module includes a wideband 90° phase shifter, a zero-crossing comparator, a circuit composed of exclusive-OR gate and OR gate and an optical clock signal output module. The interference module adopts an all-fiber Mach-Zehnder Interferometer (MZI) structure which is mainly consists of two fiber couplers. A second coupler is a symmetric $2\times2$ fiber coupler. First of all, light is divided into two paths at the first fiber coupler, these two paths of light respectively pass through a first fiber and a second fiber that have a fixed optical path difference, interference occurs at the second fiber coupler. The probe module, consistings of a high-speed balanced photodetector, is mainly used to convert an interference optical signal output from the interference module into an electrical signal. A part of the MZI electrical signal converted by the detector module is transmitted to the wideband 90° phase shifter and the other part is transferred to the zero-crossing comparator, the electrical signal transmitted to the wideband 90° phase shifter phase shift for 90°, the zero-crossing comparator is mainly used for the zero-crossing comparison of the signals undergo phase shift and with phase shift and convert them to digital signals. The exclusive-OR gate is mainly used to merge two digital clock signals to generate two clock signals in a free spectral range (FSR). In this way, a maximum imaging depth of OCT is increased without increasing the FSR, and jitter generated by the optical signal is reduced as well. In addition, because a sweep laser always has some idle time between two adjacent times of scanning, the optical clock signal needs to fill in some fake clock signals in the blank by using an OR gate to ensure that a high-speed analog-digital capture cards can work normally. The OR gate implements a function of merging real optical clock signals and fake clock signals. The optical clock signal output module is mainly used to transport the merged actual optical clock signals and real clock signals to the data collection module.

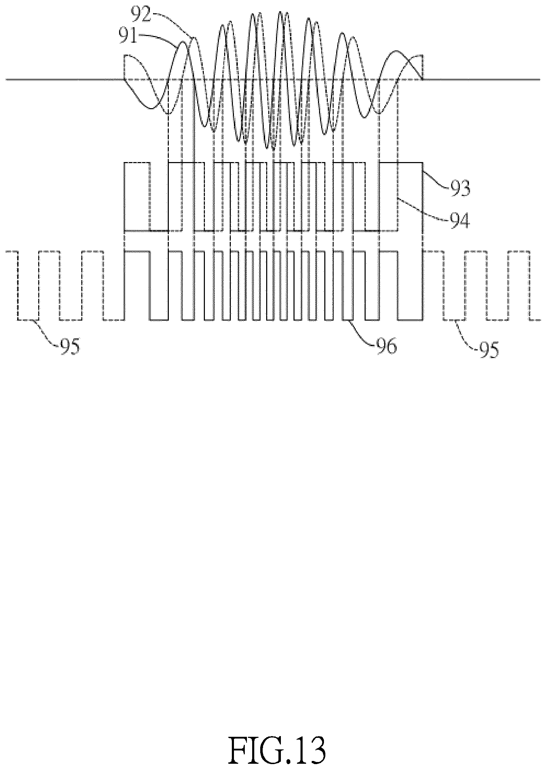

As shown in Fig. 13, 91 is a MZI electrical signal that undergo 90 degree phase shift after the wideband 90 degree phase shifter; 92 is a MZI electrical signal without a phase shift; 93 is a digital signal after the zero-crossing comparison of MZI signals from 91 which undergo phase shift; 94 is a digital signal after the zero-crossing comparison of MZI signals from 92 which is without phase shift. Since the zero points of the MZI signal are uniformly distributed on a frequency domain, rising edges or falling edges of the digital signals generated after zero crossing comparison are also uniformly distributed on a frequency domain. 96 is the signal after the digital signal 93 and 94 which are uniformly distributed on a frequency domain are merged by an exclusive OR gate, 95 is fake clock signal, 95 and 96 form the optical clock signal after the OR merge together.

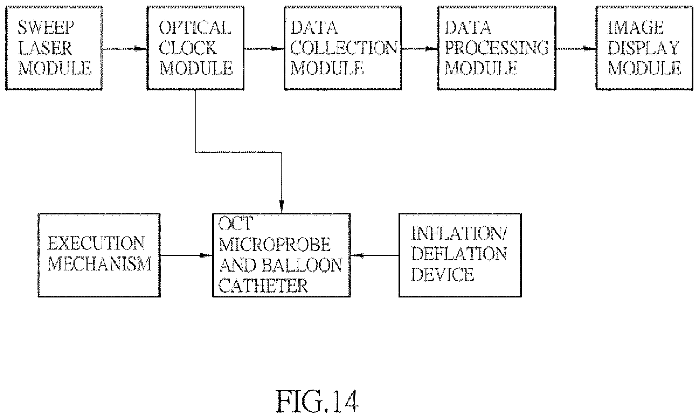

As shown in Fig. 14, an OCT endoscopic scanning and imaging system which is similar with embodiment 2-4, the difference that it comprises sweep laser module, an optical clock module, a data collection module, a data processing module, an image display module, an actuator, an OCT microprobe, a balloon catheter, and an automatic inflation/deflation device, wherein the optical clock module as shown in FIG 12, comprises an interference module, a detector module and an optical clock conversion circuit module. The optical clock conversion circuit module includes a wideband 90-degree phase shifter, zero-crossing comparators, a circuit composed of exclusive-OR gate and OR gate and an optical clock signal output module. The optical interference signal generated by the MZI is converted into an electrical signal by the balanced photodetector, and then converted into an optical clock signal that is uniform in the frequency domain and frequency-variable in time domain by an circuit module composed of the wideband 90-degree phase shifter, zero-crossing comparators, an exclusive-OR gate. The OR gate merges the real optical clock signals and fake clock signals to ensure that a high-speed analog-digital capture cards can work normally.

The sweep laser module comprises a high-speed sweep laser, a fiber isolator, a fiber couple, it stops the optical signal from the sweep laser and the subsequent optical path to prevent the optical signal of the subsequent optical path from interfering the normal operation of the laser; a small part of the sweep laser is output into the optical clock module, most of the laser continue to output. The optical clock module consists of the interference module, the probe module, and an optical clock conversion circuit module. It is mainly used to be converted into an optical clock signal that is uniform in the frequency domain and frequency-variable in time domain. The data collection module can be a high-speed analog-digital collection card and is mainly used to collect the original image signal based on the optical clock signal output from the optical clock module, and then the original image signal provided to the data processing module for processing. The data processing module is a chip (such as a CPU, a GPGPU, a DSP, or an FPGA) having a digital signal processing capability and is mainly used to process an original signal and convert the original signal into a final image signal. The image display module is mainly used to display the image signal and is responsible for post-processing and measurement of the image. The execution mechanism is formed by a fiber rotation connector, a motor, and a motorized translation stage and is mainly used to drive the OCT microprobe to perform mechanical and spiral scanning to obtain an OCT image. The OCT microprobe is mainly used to enter an internal organ of a human body to transmit sweep laser and collect an optical signal scattered backwards from tissues. The balloon catheter is used to expand tracts of internal organs of a human body, eliminate folds, and stabilize the OCT microprobe at the center of the balloon. The inflation/deflation device is mainly used to expand the balloon catheter.

Embodiment 6

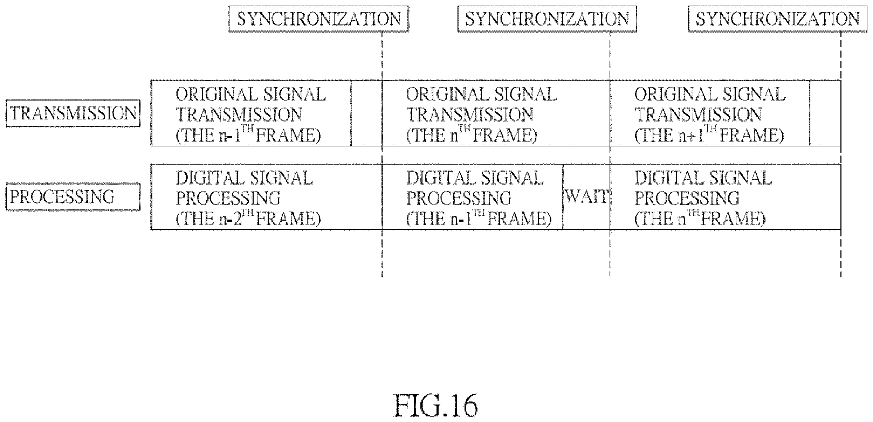

An OCT endoscopic scanning and imaging system is similar to Embodiment 2-5. The difference between the OCT endoscopic scanning and imaging system and Embodiment 2-5 is the method for processing OCT signals using GPGPU as shown in Fig.10 including the steps: (1) data collection; (2) data transmission; (3) data processing; and (4) transferring the data to an image display library. Because a transmission speed of the bus is relatively slow, at the same time of data transmission, the GPGPU performs parallel processing on the OCT original data which is previously transmitted to the device memory. The parallel processing shown in Fig.16; the digital signal processing is divided into three steps: one-dimensional digital resampling, one-dimensional Fast Fourier Transform (FFT), and amplitude calculation and normalization. In the one-dimensional digital resampling step, a quick one-dimensional cubic interpolation is implemented once by searching for linear texture twice to improve the accuracy of resampling.

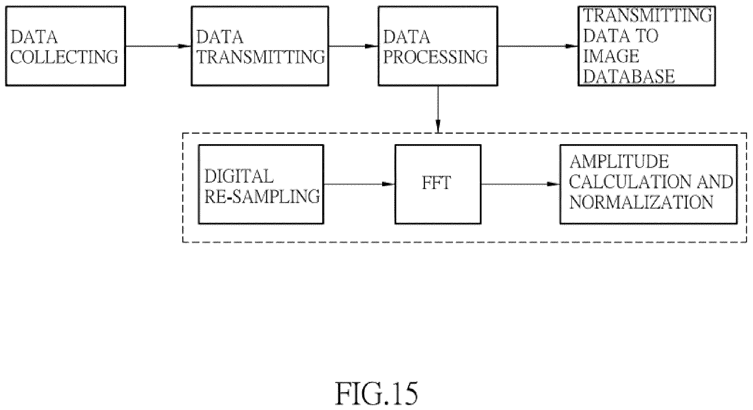

As shown in Fig. 15, a method for processing OCT signals using GPGPU including the steps: (1) data collection; (2) data transmission; (3) data processing; and (4) transferring the data to an image display library.

(1) Data collection: FD-OCT original data is obtained by using an external collection device in the present invention.

(2) Data transmission: the FD-OCT original data obtained in the data collection step is placed in a computer system or an embedded system memory. The data is stored in a system memory in frame as a unit. After a certain condition is satisfied (for example, data accumulates to one frame or multiple frames), the data can be transmitted to a device memory of the General Purpose Graphics Processing Unit (GPGPU) by using a data bus (such as PCI Express). Because a transmission speed of the bus is relatively slow, at the same time of data transmission, the GPGPU performs parallel processing on the OCT original data which is previously transmitted to the device memory.

For example, as shown in Fig.16, when the original data of the n-th frame is transferred to the memory of the general-purpose GPGPU, the original data of the (n-1) -th frame is subjected to digital signal processing in the general-purpose GPGPU at the same time. And the frame synchronization is performed after the data transmission and processing are completed, that is, whether the data is transmitted or the data processing is completed, the one of the party will wait for another completion after continuing the next frame of operation, and the parallel signal transmission / processing model can be valid improve the data processing speed of the general-purpose GPGPU.

(3) Data processing as shown in Fig.15, digital signal processing in the GPGPU is divided into three steps: one-dimensional digital resampling, one-dimensional Fast Fourier Transform (FFT), and amplitude calculation and normalization. The digital resampling can be realized by the internal texture lookup function of the Graphics Processing Unit (GPU). The internal texture lookup function of the GPU can automatically realize the two-dimensional linear interpolation, and the texture processor of the GPU has special hardware optimization, which is faster than the general image processor interpolation, especially for OCT signal processing in the non-equidistant interpolation; by setting a precision on the search point, one-dimensional linear interpolation can be realized through the internal texture search function of the GPU. On the basis of this, using texture lookup for twice can quickly achieve fast one-dimensional cubic interpolation twice, so as to improve the accuracy of resampling. As the texture search module is optimized for the non-equidistant interpolation, the calculation amount is smaller and the calculation efficiency is higher than the direct cubic interpolation on a GPGPU in this method. FFT can be implemented through a common commercial image processor-based numerical calculation library (such as nVidia's cuFFT library or OpenCL FFT library). Calculation of the amplitude and normalization can be achieved through the preparation of their own image processor program. For example, fast traversal of two-dimensional data to achieve the amplitude and normalization can be realize by using the CUDA library provided by nVidia for writing the corresponding kernel function.

(4) Transferring the data to an image display library: the processed data is placed in a memory of the image display library; the image display library can directly call the data which does not need to be transmitted by using a bus, thereby greatly improving the transmission efficiency and saving bus resources, providing an efficient parallel signal processing capability, implementing real-time digital signal processing, providing high transplantability, improving the flexibility of software display because of seamless combination with the popular image display library (for example, post-processing the image by using the GPGPU), and implementing relatively low cost of hardware and software development.

Embodiment 7

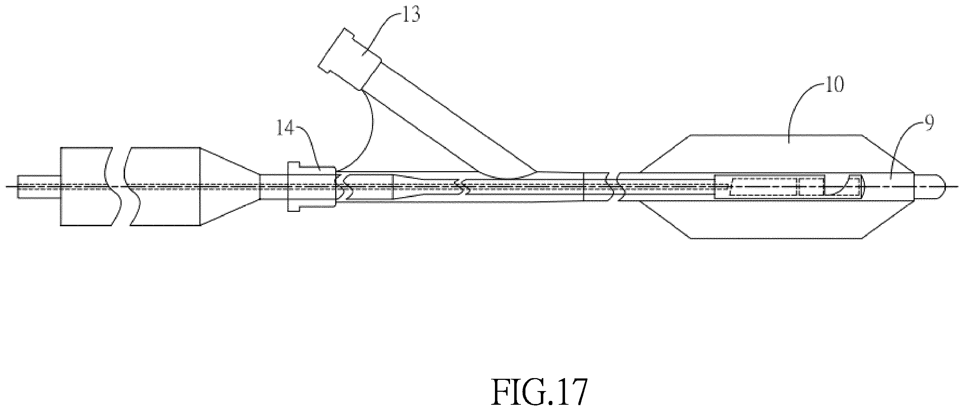

As shown in FIG. 17, in a microprobe for OCT imaging scanning in a human body esophagus, the microprobe is inserted from a near-end handle guide wire cavity of a balloon into a balloon inner tube 9. A balloon handle ventilation interface 13 is connected to an automatic air pump (not shown in the figure). The balloon 10 is inflated to a rated air pressure, so as to expand an esophagus. The balloon 10 and the inner tube 9 are both made of optical transparent materials which have extremely good light transmission performances. The balloon 10 functions to expand the esophagus to reduce esophagus folds and fix the OCT microprobe within a working distance range thereof. The radius of expansion of the balloon 10 is approximately in a range from 8 mm to 10 mm, which is also a radius when the esophagus is completely expanded. Therefore, a relatively long working distance (approximately 8 mm to 10 mm) is a characteristic that the OCT microprobe needs to have. The OCT microprobe needs to select a relatively long self-focusing lens when the working distance is relatively long. The relatively long self-focusing lens is not easily bent and may cause much inconvenience when used within a body cavity, may be cracked when bent excessively, and has a hidden danger. Therefore, the working distance of the self-focusing lens is changed by disposing the glass rod, so that the self-focusing lens becomes a multi-section structure, thereby ensuring that the microprobe has a better over-bending property, so that the entire microprobe can still enter a narrow channel when the working distance is relatively long.

The glass rod 3 is especially designed in this embodiment. It is mentioned above that the working distance of the microprobe can be changed by changing the gluing distances of the glass rod 3 and the single-mode fiber 1. The microprobe in this embodiment is used in a human body esophagus. The working distance is approximately 8 mm to 10 mm. By means of calculation and tests, the gluing distances of two surfaces of the glass rod 3 and the single-mode fiber 1 should be less than 0.3 mm. The glass rod 3 in the application not only enable the OCT probe to work within a relatively long working distance but also change the numerical aperture and the transverse resolution of the microprobe, so as to increase the numerical aperture and improve the transverse resolution of the microprobe while changing the gluing distances of the glass rod and the single-mode fiber and increasing the working distance.

A relationship between the numerical aperture and a clear aperture of an optical element is as follows:

$$N.A. = \frac{D/2}{W.D.}$$

Wherein D is the clear aperture of the optical element, W.D is the working distance, and N.A is the numerical aperture. When the working distance W.D. is fixed, the numerical aperture is in direct proportion to the clear aperture (D) of the optical element. Because of defects of the processing process of the self-focusing lens, the clear aperture is only approximately 80% of the diameter thereof. However, because the single-mode fiber is relatively thin in an actual application, the clear aperture actually used by the self-focusing lens directly connected to the single-mode fiber is only less than 10% of the diameter of the self-focusing lens. To increase the clear aperture actually used by the self-focusing lens, the glass rod is added in this application. Because light is diffusely transmitted in the glass rod, the glass rod implements a beam expansion function for light rays, thereby increasing the clear aperture actually used when the fiber passes through the self-focusing lens.

A relationship between the resolution and the numerical aperture is as follows:

$$\Delta x \ (\text{transverse resolution}) \propto \frac{\lambda}{N.A.}$$

Wherein, $\lambda$ is an incident light wavelength and is a fixed value. The transverse resolution $\Delta X$ is in direct proportion to the numerical aperture (N.A), that is, a larger numerical aperture indicates a higher transverse resolution (a smaller value).

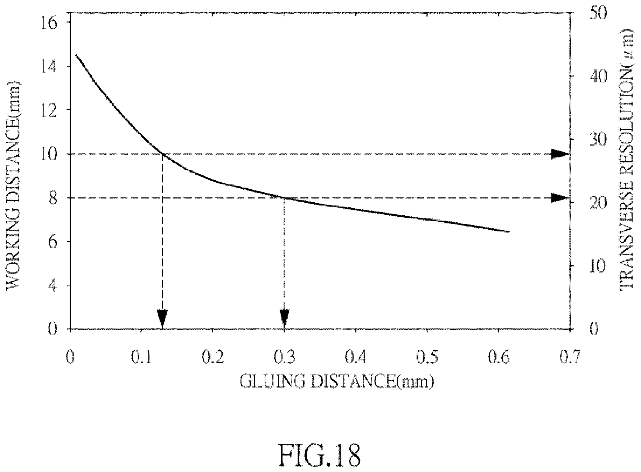

In conclusion, the use of the glass rod 3 not only increases the working distance of the microprobe, but also increases the numerical aperture of the microprobe. The increase in the numerical aperture also increases the transverse resolution. In addition, this design also greatly shortens the length of the self-focusing lens and ensures the over-bending property of the microprobe. In this way, the entire microprobe still can directly enter a human body esophagus together with the balloon catheter by using an endoscopic pliers channel. This effective design enables the transverse resolution of the probe to reach approximately 10 to 30 micrometers, and enables the working distance to reach 8 mm to 10 mm. A relationship between the working distance and the transverse resolution of the microprobe is shown in FIG. 18. The diameter of the entire microprobe is less than 1.5 mm. If a grin lens with a diameter being 1.0 mm is used, the diameter of the entire microprobe is less than 1.3 mm. If a grin lens with a diameter being 0.7 mm is used, the diameter of the entire microprobe is less than 1.0 mm. If a grin lens with a diameter being 0.5 mm is used, the diameter of the entire microprobe is less than 0.7 mm. This application enables the OCT microprobe to be applied to a narrow space and have a relatively long working distance, a relatively large numerical aperture, and a relatively high resolution.

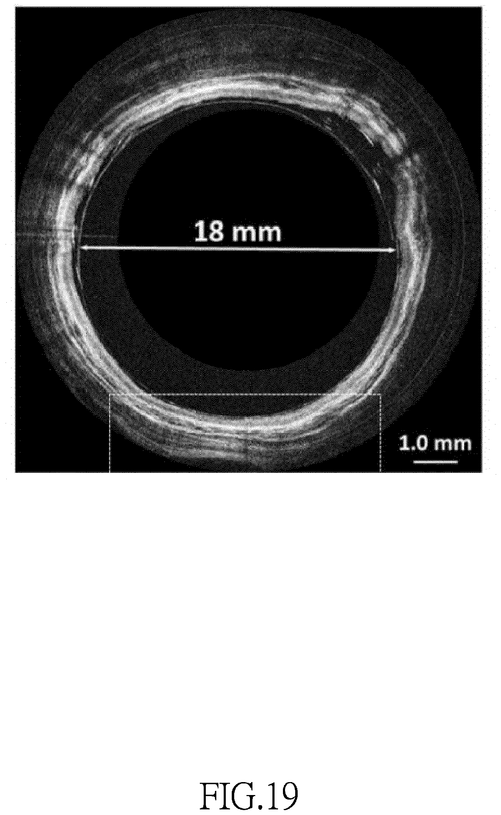

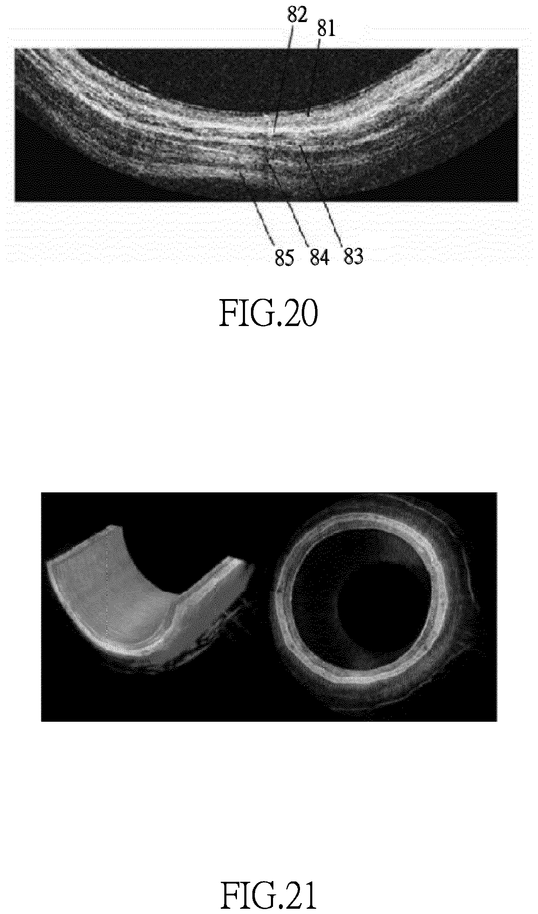

FIG. 19 shows an image of a section of an esophagus of a healthy animal, which is obtained by using an OCT balloon catheter endoscope, The size of the image is 1200 transverse scanning number×4096 longitudinal scanning, a scanning speed is 0.2 cm/3s, and a scale is 1 mm. FIG. 20 is a partial enlarged view of the image of the esophagus of the healthy animal of FIG. 19. Recognizable layers include: 81: scaly epithelial (SE) layer, 82: lamina propria (LP) layer, 83: muscularis mucosa (MM), 84: submucosa (SM), and 85: muscularis propria (MP) layer. FIG. 21 is a 3D image, generated after the OCT endoscopic scanning and imaging system performs lumen surface and depth scanning and then performs software reestablishment on scanned data, of an esophagus of a healthy animal.

The foregoing descriptions are merely preferred implementations of this application and enable a person skilled in the art to understand or implement the invention of this application. Multiple modifications and combinations of these embodiments are obvious to a person skilled in the art. A general principle defined in this text can be implemented in other embodiments without departing from the scope of this application. Therefore, this application is not limited to these embodiments shown in this text but needs to conform with a broadest scope consistent with the principle and novel features disclosed in this text. The present invention is defined by the appended claims.

**Claims**

1. An optical coherence tomography, OCT, endoscopic scanning and imaging system, comprising a balloon (10), a balloon catheter, an OCT microprobe, and an automatic inflation/deflation device, wherein the automatic inflation/deflation device comprises:

   a control and display module for setting and controlling an inflation/deflation status of the balloon (10),
   an air pump (101) that is connected to the control and display module and is controlled by the control and display module for activation and stop;
   an inflation pipeline connected between the air pump (101) and the balloon (10), wherein the air pump (101) is communicated to the balloon (10) via the inflation pipeline so as to perform inflation/deflation on the balloon (10);
   an inflation solenoid valve (102) that is provided in the inflation pipeline for starting/stopping an inflation process, wherein the inflation solenoid valve (102) is further connected to the control and display module so as to be opened/closed by the control and display module;
   a deflation solenoid valve (107) that is provided in a bypath of the inflation pipeline for starting/stopping a deflation process, wherein the deflation solenoid valve (107) is further connected to the control and display module so as to be opened/closed by the control and display module; and
   a pressure sensor (103) that is communicated with the inflation pipeline for collecting pressure data of the balloon (10), wherein the pressure sensor (103) is connected to the control and display module such that the collected data may be transmitted to the control and display module.

2. The OCT endoscopic scanning and imaging system according to claim 1, **characterized in that** the automatic inflation/deflation device further comprises a throttle valve (104) that is provided in the inflation pipeline between the inflation solenoid valve (102) and the balloon (10).

3. The OCT endoscopic scanning and imaging system according to claim 2, **characterized in that** the automatic inflation/deflation device further comprises a mechanical pressure switch (108) that is provided in the inflation pipeline between the throttle valve (104) and the balloon (10) for hardware pressure relief protection.

4. The OCT endoscopic scanning and imaging system according to claim 2, **characterized in that** the OCT endoscopic scanning and imaging system further comprises a further pressure sensor (105) that is provided in the inflation pipeline for collecting air pressure data in the balloon (10) at multiple positions.

5. The OCT endoscopic scanning and imaging system according to claim 2, **characterized in that** the OCT endoscopic scanning and imaging system further comprises an anti-explosion pressure sensor (106) that is provided in the inflation pipeline between the throttle valve (104) and the balloon (10) for detecting air pressure in the balloon (10).

6. The OCT endoscopic scanning and imaging system according to claim 4, **characterized in that** the pressure sensor (103) is provided in the inflation pipeline between the inflation solenoid valve (102) and the throttle valve (104), and the further pressure sensor (105) is provided in the inflation pipeline between the balloon (10) and the throttle valve (104).

7. The OCT endoscopic scanning and imaging system according to claim 1, **characterized in that** the OCT endoscopic scanning and imaging system further comprises a double cavity tube (11) and a handle (12), wherein the double cavity tube (11) is connected to the handle (12) at one end, and to the balloon (10) ans an inner tube (9) for passing through the OCT microprobe at the other end.

8. The OCT endoscopic scanning and imaging system according to claim 7, **characterized in that** the inner tube (9) and the balloon (10) are concentrically arranged such that an offset distance between a center of the inner tube (9) and a center of the balloon (10) is of no more than 500 micrometers under 3 atmospheric pressure.

9. The OCT endoscopic scanning and imaging system according to claim 7, **characterized in that** the OCT endoscopic scanning and imaging system further comprises an image display module and a data processing module connected to the image display module;
wherein the handle (12) is connected to a host interface (14) and a ventilation interface (13), and the air pump (101) is communicated with the inflation pipeline via the ventilation interface (13).

10. The OCT endoscopic scanning and imaging system according to claim 1, **characterized in that** the OCT microprobe comprises a single-mode fiber (1), a spring tube (2), a glass rod (3), a self-focusing lens (4) and a reflector (5);

wherein the single-mode fiber (1) is arranged in the spring tube (2) for transmitting light signals and torsion force; one end of the glass rod (3) is surface-glued to the self-focusing lens (4) at a zero angle, and the other end of the glass rod (3) is obliquely surface-glued to the single-mode fiber (1);
wherein the reflector (5) is provided at a side of the self-focusing lens (4) that is far away from the glass rod (3).

11. The OCT endoscopic scanning and imaging system according to claim 1, **characterized in that** the control and display module is set for performing procedures of:

an inflation process that sets an inflation pressure and an inflation time of the balloon (10), and activates the air pump (101) and the inflation solenoid valve (102) to inflate the balloon (10) until a preset inflation pressure is reached;
a deflation process that sets a deflation pressure and a deflation time of the balloon (10), and activates the air pump (101) and the deflation solenoid valve (107) to deflate the balloon (10) until a preset deflation pressure is reached.

12. The OCT endoscopic scanning and imaging system according to claim 11, **characterized in that**

the endoscopic scanning and imaging system further comprises an anti-explosion pressure sensor (106) that is provided in the inflation pipeline between the throttle valve (104) and the balloon (10) for detecting air pressure in the balloon (10), and
the control and display module is further set for performing procedures of:
the air pump (101) being turned off when the air pressure monitored by the anti-explosion pressure sensor (106) exceeds a preset pressure, and/or the pressure is relieved through a mechanical pressure switch (108) communicated with the balloon (10).

**Patentansprüche**

1. Endoskopisches Abtast- und Bildgebungssystem für optische Kohärenztomographie, OCT, das einen Ballon (10), einen Ballonkatheter, eine OCT-Mikrosonde und eine automatische Aufblas-/Entleerungsvorrichtung umfasst, wobei die automatische Aufblas-/Entleerungsvorrichtung Folgendes umfasst:

ein Steuer- und Anzeigemodul zum Einstellen und Steuern eines Aufblas-/Entleerungsstatus des Ballons (10),
eine Luftpumpe (101), die mit dem Steuer- und Anzeigemodul verbunden ist und vom Steuer- und Anzeigemodul zum Aktivieren und Beenden gesteuert wird;
eine Aufblasleitung, die zwischen der Luftpumpe (101) und dem Ballon (10) verbunden ist, wobei die Luftpumpe (101) via die Aufblasleitung mit dem Ballon (10) kommuniziert, um ein Aufblasen/Entleeren des Ballons (10) durchzuführen;

ein Aufblasmagnetventil (102), das zum Starten/Beenden eines Aufblasprozesses in der Aufblasleitung bereitgestellt ist, wobei das Aufblasmagnetventil (102) ferner mit dem Steuer- und Anzeigemodul verbunden ist, um vom Steuer- und Anzeigemodul geöffnet/geschlossen zu werden;

ein Entleerungsmagnetventil (107), das zum Starten/Beenden eines Entleerungsprozesses in einem Seitenpfad der Aufblasleitung bereitgestellt ist, wobei das Entleerungsmagnetventil (107) ferner mit dem Steuer- und Anzeigemodul verbunden ist, um vom Steuer- und Anzeigemodul geöffnet/geschlossen zu werden; und

einen Drucksensor (103), der zum Sammeln von Druckdaten des Ballons (10) mit der Aufblasleitung kommuniziert, wobei der Drucksensor (103) mit dem Steuer- und Anzeigemodul verbunden ist, derart, dass die gesammelten Daten zum Steuer- und Anzeigemodul übertragen werden können.

2. Endoskopisches OCT-Abtast- und Bildgebungssystem nach Anspruch 1, **dadurch gekennzeichnet, dass** die automatische Aufblas-/Entleerungsvorrichtung ferner ein Drosselventil (104) umfasst, das in der Aufblasleitung zwischen dem Aufblasmagnetventil (102) und dem Ballon (10) bereitgestellt ist.

3. Endoskopisches OCT-Abtast- und Bildgebungssystem nach Anspruch 2, **dadurch gekennzeichnet, dass** die automatische Aufblas-/Entleerungsvorrichtung ferner einen mechanischen Druckschalter (108) umfasst, der zum Druckabbauschutz der Hardware in der Aufblasleitung zwischen dem Drosselventil (104) und dem Ballon (10) bereitgestellt ist.

4. Endoskopisches OCT-Abtast- und Bildgebungssystem nach Anspruch 2, **dadurch gekennzeichnet, dass** das OCT-Abtast- und Bildgebungssystem ferner einen weiteren Drucksensor (105) umfasst, der zum Sammeln von Luftdruckdaten im Ballon (10) an mehreren Stellen in der Aufblasleitung bereitgestellt ist.

5. Endoskopisches OCT-Abtast- und Bildgebungssystem nach Anspruch 2, **dadurch gekennzeichnet, dass** das OCT-Abtast- und Bildgebungssystem ferner einen Antiexplosionsdrucksensor (106) umfasst, der zum Detektieren eines Luftdrucks im Ballon (10) in der Aufblasleitung zwischen dem Drosselventil (104) und dem Ballon (10) bereitgestellt ist.

6. Endoskopisches OCT-Abtast- und Bildgebungssystem nach Anspruch 4, **dadurch gekennzeichnet, dass** der Drucksensor (103) in der Aufblasleitung zwischen dem Aufblasmagnetventil (102) und dem Drosselventil (104) bereitgestellt ist, und der weitere Drucksensor (105) in der Aufblasleitung zwischen dem Ballon (10) und dem Drosselventil (104) bereitgestellt ist.

7. Endoskopisches OCT-Abtast- und Bildgebungssystem nach Anspruch 1, **dadurch gekennzeichnet, dass** das OCT-Abtast- und Bildgebungssystem ferner ein Doppelhohlraumrohr (11) und einen Griff (12) umfasst, wobei das Doppelhohlraumrohr (11) an einem Ende mit dem Griff (12) und am anderen Ende mit dem Ballon (10) und einem inneren Rohr (9) zum Durchführen durch die OCT-Mikrosonde verbunden ist.

8. Endoskopisches OCT-Abtast- und Bildgebungssystem nach Anspruch 7, **dadurch gekennzeichnet, dass** das innere Rohr (9) und der Ballon (10) konzentrisch angeordnet sind, derart, dass ein Versatzabstand zwischen einer Mitte des inneren Rohrs (9) und einer Mitte des Ballons (10) unter einem Atmosphärendruck von 3 nicht mehr als 500 Mikrometer beträgt.

9. Endoskopisches OCT-Abtast- und Bildgebungssystem nach Anspruch 7, **dadurch gekennzeichnet, dass** das OCT-Abtast- und Bildgebungssystem ferner ein Bildanzeigemodul und ein Datenverarbeitungsmodul, das mit dem Bildanzeigemodul verbunden ist, umfasst;

wobei der Griff (12) mit einer Hostschnittstelle (14) und einer Entlüftungsschnittstelle (13) verbunden ist, und die Luftpumpe (101) via die Entlüftungsschnittstelle (13) mit der Aufblasleitung kommuniziert.

10. Endoskopisches OCT-Abtast- und Bildgebungssystem nach Anspruch 1, **dadurch gekennzeichnet, dass** die OCT-Mikrosonde eine Monomodefaser (1), ein Federrohr (2), einen Glasstab (3), eine Selbstfokussierungslinse (4) und einen Reflektor (5) umfasst;

wobei die Monomodefaser (1) zum Übertragen von Lichtsignalen und einer Torsionskraft im Federrohr (2) angeordnet ist; ein Ende des Glasstabs (3) in einem Winkel von null an eine Oberfläche der Selbstfokussierungslinse (4) geklebt ist, und das andere Ende des Glasstabs (3) schräg an eine Oberfläche der Monomodefaser (1) geklebt ist;

wobei der Reflektor (5) an einer vom Glasstab (3) abgewandten Seite der Selbstfokussierungslinse (4) bereitgestellt ist.

**11.** Endoskopisches Abtast- und Bildgebungssystem nach Anspruch 1, **dadurch gekennzeichnet, dass** das Steuer- und Anzeigemodul zum Durchführen der folgenden Prozeduren eingestellt ist:

einen Aufblasprozess, der einen Aufblasdruck und eine Aufblaszeit des Ballons (10) einstellt, und die Luftpumpe (101) und das Aufblasmagnetventil (102) zum Aufblasen des Ballons (10) aktiviert, bis ein voreingestellter Aufblasdruck erreicht ist;

einen Entleerungsprozess, der einen Entleerungsdruck und eine Entleerungszeit des Ballons (10) einstellt, und die Luftpumpe (101) und das Entleerungsmagnetventil (107) zum Entleeren des Ballons (10) aktiviert, bis ein voreingestellter Entleerungsdruck erreicht ist.

**12.** Endoskopisches Abtast- und Bildgebungssystem nach Anspruch 11, **dadurch gekennzeichnet, dass**

das endoskopische Abtast- und Bildgebungssystem ferner einen Antiexplosionsdrucksensor (106) umfasst, der zum Detektieren eines Luftdrucks im Ballon (10) in der Aufblasleitung zwischen dem Drosselventil (104) und dem Ballon (10) bereitgestellt ist, und

das Steuer- und Anzeigemodul ferner zum Durchführen der folgenden Prozeduren eingestellt ist:

Ausschalten der Luftpumpe (101), wenn der vom Antiexplosionsdrucksensor (106) überwachte Luftdruck einen voreingestellten Druck überschreitet, und/oder der Druck über einen mechanischen Druckschalter (108), der mit dem Ballon (10) kommuniziert, abgebaut wird.

## Revendications

**1.** Système de balayage et d'imagerie endoscopique pour tomographie par cohérence optique, OCT, comprenant un ballonnet (10), un cathéter à ballonnet, une microsonde OCT et un dispositif de gonflage/dégonflage automatique, dans lequel :

le dispositif de gonflage/dégonflage automatique comprend :

un module de commande et d'affichage pour déterminer et commander un état de gonflage/dégonflage du ballonnet (10),

une pompe à air (101) qui est raccordée au module de commande et d'affichage et est commandée par le module de commande et d'affichage pour l'activation et l'arrêt ;

une conduite de gonflage raccordée entre la pompe à air (101) et le ballonnet (10), dans lequel la pompe à air (101) est en communication avec le ballonnet (10) via la conduite de gonflage afin de réaliser le gonflage/dégonflage sur le ballonnet (10) ;

une électrovanne de gonflage (102) qui est prévue dans la conduite de gonflage pour activer/arrêter un processus de gonflage, dans lequel l'électrovanne de gonflage (102) est en outre raccordée au module de commande et d'affichage afin d'être ouverte/fermée par le module de commande et d'affichage ;

une électrovanne de dégonflage (107) qui est prévue dans une dérivation de la conduite de gonflage pour activer/arrêter un processus de dégonflage, dans lequel l'électrovanne de gonflage (107) est en outre raccordée au module de commande et d'affichage pour être ouverte/fermée par le module de commande et d'affichage ; et

un capteur de pression (103) qui est en communication avec la conduite de gonflage pour collecter des données de pression du ballonnet (10), dans lequel le capteur de pression (103) est raccordé au module de commande et d'affichage de sorte que les données collectées peuvent être transmises au module de commande et d'affichage.

**2.** Système de balayage et d'imagerie endoscopique OCT selon la revendication 1, **caractérisé en ce que** le dispositif de gonflage/dégonflage automatique comprend en outre une valve d'étranglement (104) qui est prévue dans la conduite de gonflage entre l'électrovanne de gonflage (102) et le ballonnet (10).

**3.** Système de balayage et d'imagerie endoscopique OCT selon la revendication 2, **caractérisé en ce que** le dispositif de gonflage/dégonflage automatique comprend en outre un commutateur de pression mécanique (108) qui est prévu dans la conduite de gonflage entre la valve d'étranglement (104) et le ballonnet (10) pour la protection matérielle de décharge de pression.

**4.** Système de balayage et d'imagerie endoscopique OCT selon la revendication 2, **caractérisé en ce que** le système de balayage et d'imagerie endoscopique OCT comprend en outre un autre capteur de pression (105) qui est prévu dans la conduite de gonflage pour collecter des données de pression d'air dans le ballonnet (10) dans plusieurs positions.

**5.** Système de balayage et d'imagerie endoscopique OCT selon la revendication 2, **caractérisé en ce que** le système de balayage et d'imagerie endoscopique OCT comprend en outre un capteur de pression anti-explosion (106) qui est prévu dans la conduite de gonflage entre la valve d'étranglement (104) et le ballonnet (10) pour détecter la pression d'air dans le ballonnet (10) .

6. Système de balayage et d'imagerie endoscopique OCT selon la revendication 4, **caractérisé en ce que** le capteur de pression (103) est prévu dans la conduite de gonflage entre l'électrovanne de gonflage (102) et la valve d'étranglement (104) et l'autre capteur de pression (105) est prévu dans la conduite de gonflage entre le ballonnet (10) et la valve d'étranglement (104).

7. Système de balayage et d'imagerie endoscopique OCT selon la revendication 1, **caractérisé en ce que** le système de balayage et d'imagerie endoscopique OCT comprend en outre un tube à double cavité (11) et une poignée (12), dans lequel le tube à double cavité (11) est raccordé à la poignée (12) au niveau d'une extrémité et au ballonnet (10) et à un tube interne (9) pour passer par la microsonde OCT au niveau de l'autre extrémité.

8. Système de balayage et d'imagerie endoscopique OCT selon la revendication 7, **caractérisé en ce que** le tube interne (9) et le ballonnet (10) sont agencés, de manière concentrique, de sorte qu'une distance de décalage entre un centre du tube interne (9) et un centre du ballonnet (10) n'est pas supérieure à 500 micromètres à une pression atmosphérique de 3.

9. Système de balayage et d'imagerie endoscopique OCT selon la revendication 7, **caractérisé en ce que** le système de balayage et d'imagerie endoscopique OCT comprend en outre un module d'affichage d'image et un module de traitement de données raccordé au module d'affichage d'image ;
dans lequel la poignée (12) est raccordée à une interface hôte (14) et à une interface de ventilation (13), et la pompe à air (101) est en communication avec la conduite de gonflage via l'interface de ventilation (13).

10. Système de balayage et d'imagerie endoscopique OCT selon la revendication 1, **caractérisé en ce que** la microsonde OCT comprend une fibre monomode (1), un tube à ressort (2), une tige de verre (3), une lentille à auto-focalisation (4) et un réflecteur (5) ;

dans lequel la fibre monomode (1) est agencée dans le tube à ressort (2) pour transmettre des signaux de lumière et une force de torsion ; une extrémité de la tige de verre (3) est collée en surface sur la lentille à auto-focalisation (4) à un angle nul, et l'autre extrémité de la tige de verre (3) est collée obliquement en surface sur la fibre monomode (1) ;
dans lequel le réflecteur (5) est prévu sur un côté de la lentille à auto-focalisation (4) qui est éloignée de la tige de verre (3).

11. Système de balayage et d'imagerie endoscopique OCT selon la revendication 1, **caractérisé en ce que** le module de commande et d'affichage est déterminé pour réaliser les procédures suivantes :

un processus de gonflage qui détermine une pression de gonflage et un temps de gonflage du ballonnet (10) et active la pompe à air (101) et l'électrovanne de gonflage (102) pour gonfler le ballonnet (10) jusqu'à ce que la pression de gonflage prédéterminée soit atteinte ;
un processus de dégonflage qui détermine une pression de dégonflage et un temps de dégonflage du ballonnet (10) et active la pompe à air (101) et l'électrovanne de dégonflage (107) pour dégonfler le ballonnet (10) jusqu'à ce que la pression de dégonflage prédéterminée soit atteinte.

12. Système de balayage et d'imagerie endoscopique OCT selon la revendication 11, **caractérisé en ce que** :

le système de balayage et d'imagerie endoscopique comprend en outre un capteur de pression anti-explosion (106) qui est prévu dans la conduite de gonflage entre la valve d'étranglement (104) et le ballonnet (10) pour détecter la pression d'air dans le ballonnet (10), et
le module de commande et d'affichage est en outre déterminé pour réaliser les procédures suivantes :
la pompe à air (101) étant arrêtée lorsque la pression d'air surveillée par le capteur de pression anti-explosion (106) dépasse une pression prédéterminée et/ou la pression est libérée par un commutateur de pression mécanique (108) en communication avec le ballonnet (10).

```
┌──────────┐     ┌──────────────┐     ┌──────────────┐
│ POWER    │     │ AIR PUMP AND │     │ CONTROL      │
│ SOURCE   │────▶│ ITS CONTROL  │◀───▶│ AND DISPLAY  │
│ PART     │     │ SYSTEM       │     │ PART         │
└──────────┘     └──────────────┘     └──────────────┘
```

| AIR PUMP | INFLATION /DEFLATION SOLENOID VALVE | PRESSURE SENSORS | ANTI-EXPLOSION PRESSURE SENSOR | MECHANICAL PRESSURE SWITCH | THROTTLE VALVE |

# FIG.1

FIG.2

```
┌──────────┐   ┌──────────────┐   ┌──────────┐   ┌──────────────┐   ┌──────────────┐   ┌──────────┐
│ SWEEP    │   │ INTERFERENCE │   │ PROBE    │   │ DATA         │   │ DATA         │   │ IMAGE    │
│ LASER    │──▶│ MODULE       │──▶│ MODULE   │   │ COLLECTION   │──▶│ PROCESSING   │──▶│ DISPLAY  │
│ MODULE   │   │              │   │          │   │ MODULE       │   │ MODULE       │   │ MODULE   │
└──────────┘   └──────────────┘   └──────────┘   └──────────────┘   └──────────────┘   └──────────┘
```

```
┌──────────────┐   ┌──────────────────┐   ┌──────────────┐
│ EXECUTION    │   │ OCT MICROPROBE   │   │ NFLATION/     │
│ MECHANISM    │──▶│ AND BALLOON      │◀──│ DEFLATION     │
│              │   │ CATHETER         │   │ DEVICE        │
└──────────────┘   └──────────────────┘   └──────────────┘
```

FIG.3

FIG.4

FIG.5A

FIG.5B

EP 3 682 937 B1

FIG.6

FIG.7

FIG.8

FIG.9

FIG.10

FIG.11

FIG.12

FIG.13

FIG.14

```
┌──────────────┐     ┌──────────────┐     ┌──────────────┐     ┌──────────────┐
│ DATA         │────▶│ DATA         │────▶│ DATA         │────▶│ TRANSMITTING │
│ COLLECTING   │     │ TRANSMITTING │     │ PROCESSING   │     │ DATA TO      │
│              │     │              │     │              │     │ IMAGE        │
│              │     │              │     │              │     │ DATABASE     │
└──────────────┘     └──────────────┘     └──────────────┘     └──────────────┘
                                                 │
                                                 ▼
   ┌ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ┐
     ┌──────────────┐     ┌──────────────┐     ┌──────────────────┐
   │ │ DIGITAL      │────▶│     FFT      │────▶│ AMPLITUDE        │         │
     │ RE-SAMPLING  │     │              │     │ CALCULATION AND  │
   │ │              │     │              │     │ NORMALIZATION    │         │
     └──────────────┘     └──────────────┘     └──────────────────┘
   └ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ┘
```

FIG.15

EP 3 682 937 B1

| | SYNCHRONIZATION | SYNCHRONIZATION | SYNCHRONIZATION |
|---|---|---|---|
| TRANSMISSION | ORIGINAL SIGNAL TRANSMISSION (THE $n-1^{TH}$ FRAME) | ORIGINAL SIGNAL TRANSMISSION (THE $n^{TH}$ FRAME) | ORIGINAL SIGNAL TRANSMISSION (THE $n+1^{TH}$ FRAME) |
| PROCESSING | DIGITAL SIGNAL PROCESSING (THE $n-2^{TH}$ FRAME) | DIGITAL SIGNAL PROCESSING (THE $n-1^{TH}$ FRAME) | WAIT | DIGITAL SIGNAL PROCESSING (THE $n^{TH}$ FRAME) |

FIG.16

FIG.17

EP 3 682 937 B1

FIG.18

FIG.19

FIG.20

FIG.21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010004544 A1